# EUROPEAN PATENT SPECIFICATION

(11) **EP 3 765 088 B1**
(45) Date of publication and mention of the grant of the patent: **20.03.2024**
(21) Application number: 19767230.6
(22) Date of filing: 14.03.2019
(51) Int. Cl.: A61K 9/107, A61K 47/44, A61K 31/05, A61K 36/185, A61K 36/31, A61K 45/06, A61K 47/54, A61K 47/10, A61K 47/14

(54) **TRANSDERMAL AND/OR DERMAL DELIVERY OF LIPOPHILIC ACTIVE AGENTS**
TRANSDERMALE UND / ODER DERMALE ABGABE LIPOPHILER WIRKSTOFFE
ADMINISTRATION TRANSDERMIQUE ET/OU DERMIQUE D'AGENTS ACTIFS LIPOPHILES

(30) Priority: 14.03.2018 US 201862642737 P
(43) Date of publication of application: 20.01.2021
(73) Proprietor: Poviva Corp., Carson City, NV 89701 (US)
(72) Inventor: DOCHERTY, John, Port Perry, ON L9L 1V1 (CA); BUNKA, Christopher Andrew, Kelowna, BC V1W 5H5 (CA)
(74) Representative: Marks & Clerk LLP
(86) International application number: PCT/US2019/022278
(87) International publication number: WO 2019/178360

(56) References cited:
- EP-A2- 0 995 428
- CZ-A3- 2 010 764
- US-A1- 2005 271 596
- US-A1- 2006 013 777
- US-A1- 2017 172 977
- US-B1- 6 416 768
- WIKIPEDIA: "Hemp oil", Wikipedia, 24 October 2017 (2017-10-24), XP055636536, Retrieved from the Internet: URL:https://en.wikipedia.org/w/index.php?t itle=Hemp_oil&oldid=806917834 [retrieved on 2019-05-14]

## Description

### CROSS-REFERENCE TO RELATED APPLICATIONS

This application is a PCT International Application which claims the benefit of U.S. Provisional Application No. 62/642,737, filed March 14, 2018.

### TECHNICAL FIELD

Aspects described herein relate to improved compositions and methods for transdermal and/or dermal delivery of lipophilic active agents, particularly without the need for added penetration enhancers.

### BACKGROUND

Skin protects the body's organs from external environmental threats and acts as a thermostat to maintain body temperature. Skin consists of several different layers, each with specialized functions. The major layers include the epidermis, the dermis, and the hypodermis. The epidermis is a stratifying layer of epithelial cells, the dermis consists of connective tissue, and the hypodermis is an internal layer of adipose tissue.

The epidermis, the topmost layer of skin, is only 0.1 to 1.5 millimeters thick (Inlander, Skin, New York, N.Y.: People's Medical Society, 1-7 (1998)). The epidermis consists of keratinocytes and can be further classified into the stratum corneum and the viable epidermis. The stratum corneum is hygroscopic and requires at least 10% moisture by weight to maintain its flexibility and softness. The hygroscopicity is attributable in part to the water-holding capacity of keratin.

The dermis, which lies just beneath the epidermis, is 1.5 to 4 millimeters thick. The dermis is the thickest of the three layers of the skin. In addition, the dermis is also home to most of the skin's structures, including sweat and oil glands (which secrete substances through openings in the skin called pores), hair follicles, nerve endings, and blood and lymph vessels (Inlander, Skin, New York, N.Y.: People's Medical Society, 1-7 (1998)). However, the main components of the dermis are collagen and elastin.

The hypodermis is the deepest layer of the skin. The hypodermis acts both as an insulator for body heat conservation and as a shock absorber for organ protection (Inlander, Skin, New York, N.Y.: People's Medical Society, 1-7 (1998)). In addition, the hypodermis also stores fat for energy reserves.

The pH of skin is normally between 5 and 6. This acidity is due to the presence of amphoteric amino acids, lactic acid, and fatty acids from the secretions of the sebaceous glands. The term "acid mantle" refers to the presence of the water-soluble substances on most regions of the skin. The buffering capacity of the skin is due in part to these secretions stored in the skin's horny layer.

One of the principal functions of skin is to provide a barrier to the transportation of water and substances potentially harmful to normal homeostasis. The body would rapidly dehydrate without a tough, semi-permeable skin. The skin helps to prevent the entry of harmful substances into the body.

Delivery of drugs through the skin has been both an attractive and challenging area for research (Paudel et al. (2010) Ther. Deliv. 1(1): 109-131). Advances in modern technologies have resulted in an increasing number of drugs being delivered transdermally, including conventional hydrophobic small molecule drugs, hydrophilic drugs, and macromolecules (Paudel et al. (2010) Ther. Deliv. 1(1):109-131). Transdermal systems offer several advantages over other routes of delivery. For example, transdermal delivery avoids hepatic first-pass metabolism and the gastrointestinal tract for poorly bioavailable drugs (Paudel et al. (2010) Ther. Deliv. 1(1):109-131). Elimination of this first-pass effect allows the amount of drug administered to be lower, which can result in the reduction of adverse effects (Paudel et al. (2010) Ther. Deliv. 1(1):109-131). Another advantage of transdermal delivery is that it provides convenient and pain-free self-administration for patients (Paudel et al. (2010) Ther. Deliv. 1(1):109-131). Transdermal delivery can eliminate plasma level peaks and valleys associated with oral dosing and injections to maintain a constant drug concentration, and a drug with a short half- life can be delivered easily (Paudel et al. (2010) Ther. Deliv. 1(1):109-131). Collectively, these benefits can lead to enhanced patient compliance, especially when long-term treatment is required (Paudel et al. (2010) Ther. Deliv. 1(1):109-131).

Other advantages of transdermal and/or dermal formulations is that such formulations offer a possibility for administering medicines to patients who have difficulty in swallowing oral formulations. Such formulations also provide an alternative in cases when prolonged parenteral medication should be replaced. Furthermore, transdermal and/or dermal formulations offer the possibility of localized administration of the medicament thus reducing or preventing side effects. Such formulations are also suitable for the administration of active ingredients which are metabolized rapidly and extensively subsequent to oral administration.

A major obstacle to transdermal delivery is the excellent barrier characteristics exhibited by skin. Several routes through the skin have been identified. The transappendageal route transports substances through sweat glands and hair follicles with their sebaceous glands. This route is considered of minor importance due to the very limited area (less than 0.1% of total skin surface). However, large compounds can theoretically be delivered by this route. The transepidermal route consists of transport via diffusion through the cellular layer that includes the stratum corneum (consisting of lipids), viable epidermis (90% water held together by tonofibrils), and dermis (loose connective tissue composed of fibrous protein embedded in an amorphous ground substance).

Increased penetration of the skin barrier has been achieved by use of physical methods, biological methods, and chemical penetration enhancers. Physical methods have included iontophoresis, sonophoresis, thermal energy, and stripping of the stratum corneum. Biological approaches have included utilizing a therapeutically inactive prodrug that is transported through the skin barrier by physiological mechanisms with the prodrug then being metabolized to produce the therapeutically active drug. Chemical penetration enhancers reversibly decrease the barrier allowing drug permeation.

Therefore, there is a need for improved compositions and methods for transdermal and/or dermal administration of lipophilic active agents to treat a variety of disorders in subjects in need thereof.

EP0995428 A2 describes the stabilization of vitamin A and its derivatives by special oil-water emulsifiers in oil-water emulsions to be applied topically.

CZ2010764 A3 describes compositions for the treatment of inflammatory diseases based on natural substances.

US2005/271596 A1 relates to a therapeutic kit to provide a safe and effective dosage of a vasoactive agent, including an aerosol packaging assembly having a container accommodating a pressurized product as a foam. The pressurized product comprises a foamable composition.

### SUMMARY

To address the foregoing problems, in whole or in part, and/or other problems that may have been observed by persons skilled in the art, the present disclosure provides compositions and methods as described by way of example as set forth below. The invention is defined by the appended claims which relate to a transdermal and/or dermal composition.

The presently disclosed compositions and methods demonstrate efficacy for transdermal and/or dermal administration of active agents without the need for added penetration enhancers. However, although the presently disclosed compositions and methods do not require added penetration enhancers, such added penetration enhancers may be optionally incorporated.

A transdermal and/or dermal composition according to the invention comprises a lipophilic active agent in an oil-in-water emulsion comprising an aqueous phase and an oil phase, wherein the composition is obtainable by providing an oil phase comprising a dehydrated mixture, and wherein the dehydrated mixture comprises a therapeutically effective amount of the lipophilic active agent and an edible oil comprising long chain fatty acids and/or medium chain fatty acids, wherein the lipophilic active agent is cannabidiol and the edible oil is sunflower oil, and wherein the dehydrated mixture is obtainable by the steps of:
i) combining the therapeutically effective amount of the cannabidiol with the sunflower oil; and
ii) dehydrating the product of step (i), thereby producing the dehydrated mixture; and
combining the oil phase with an aqueous phase to provide the oil-in-water emulsion.

In some aspects, within the transdermal and/or dermal composition, the bioavailability of the lipophilic active agent in a subject is at least 2 times, 5 time, or 10 times greater than the bioavailability of the lipophilic active agent in the subject in the absence of the edible oil comprising long chain fatty acids and/or medium chain fatty acids. In some aspects, the edible oil comprising long chain fatty acids and/or medium chain fatty acids is substantially free of omega-6 fatty acids. In some aspects, the long chain fatty acids and/or medium chain fatty acids are selected from the group consisting of oleic acid, undecanoic acid, valeric acid, heptanoic acid, pelargonic acid, capric acid, lauric acid, and eicosapentaenoic acid.

The lipophilic active agent is the cannabinoid cannabidiol.

In some aspects, the transdermal and/or dermal composition further comprises an additional formulation component selected from the group consisting of Penetration Enhancers, Essential Oils, Plant Extracts, Thickening Agents, Neutralizing Agents, Wetting Agents (Surfactants), Lubricants, Emollients, Emulsifying Agents, Solubilizers, Oils and Waxes, Higher Fatty Acids, Higher Alcohols, Cosmetic Ingredients, UV Absorption Agents, Moisturizing Agents, Antioxidants, Structuring Agents, Emulsifiers, Silicone Containing Compounds, Preservatives, and Conditioning Agents.

In some aspects, a process is provided for making a transdermal and/or dermal composition comprising a lipophilic active agent in an oil-in-water emulsion comprising an aqueous phase and an oil phase, the steps comprising:
i) combining a therapeutically effective amount of the lipophilic active agent with an edible oil comprising long chain fatty acids and/or medium chain fatty acids;
ii) dehydrating the product of step (i), thereby producing a dehydrated mixture; and
iii) combining the aqueous phase with the oil phase, wherein the oil phase comprises the dehydrated mixture of step (ii).

Although not according to the invention, a method is described for treating a condition comprising administering the disclosed transdermal and/or dermal compositions to a subject in need thereof, wherein the condition is a skin condition selected from the group consisting of skin ageing, elastosis, laxity (sagging), rhytids (wrinkles), skin infection, skin damage, skin burn, pain, and muscle tightness, and combinations thereof.

Although not according to the invention, a method is described for treating a condition comprising administering the disclosed transdermal and/or dermal compositions to a subject in need thereof, wherein the lipophilic active agent is a cannabinoid and the condition is selected from the group consisting of cardiac diseases such as heart disease, ischemic infarcts, and cardiometabolic disorders; neurological diseases such as Alzheimer's disease, Parkinson's disease, schizophrenia, and Human Immunodeficiency Virus (HIV) dementia; obesity; metabolic disorders such as insulin related deficiencies and lipid profiles, hepatic diseases, diabetes, and appetite disorders; cancer chemotherapy; benign prostatic hypertrophy; irritable bowel syndrome; biliary diseases; ovarian disorders; marijuana abuse; alcohol, opioid, nicotine, or cocaine addiction; and sexual dysfunction such as erectile dysfunction.

Although not according to the invention, a kit is described comprising a transdermal and/or dermal composition and instructions for use thereof.

Other compositions, features, and advantages of the invention will be or will become apparent to one with skill in the art upon examination of the following figures and detailed description. It is intended that all such additional compositions, features, and advantages be included within this description, be within the scope of the invention, and be protected by the accompanying claims.

### BRIEF DESCRIPTION OF THE DRAWINGS

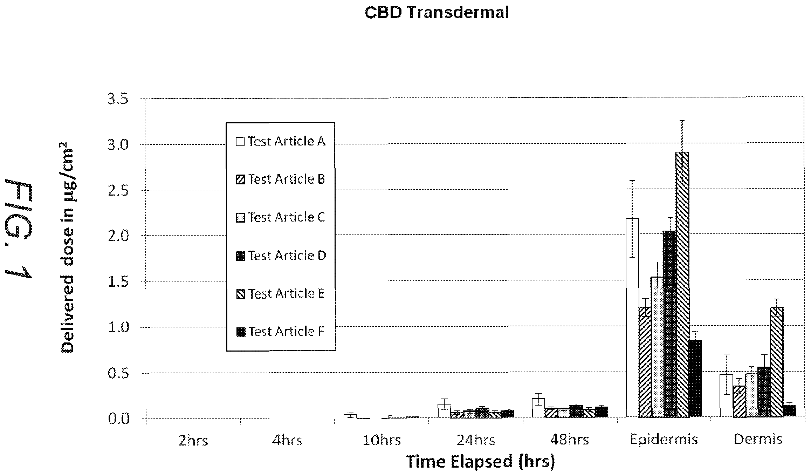

The invention can be better understood by referring to the following figures. The components in the figures are not necessarily to scale, emphasis instead being placed upon illustrating the principles of the invention. As described in more detail below, the data shown in Figures 1 to 4 are from an *in vitro* Franz diffusion cell skin permeability investigation undertaken to evaluate permeability of lipophilic active agents in the formulation of the present invention relative to a series of comparator control formulations.
Figure 1 shows transdermal delivery of CBD in µg/cm² for Test Articles A through F, at 2h, 4h, 10h, 24h, and 48 hr, as well as skin retention in the epidermis and dermis.
Figure 2 shows skin retention of CBD in µg/cm² for Test Articles A through F in the epidermis and dermis.
Figure 3 shows CBD percent delivery for Test Articles A through F, at 2h, 4h, 10h, 24h, and 48 hr, as well as in the epidermis and dermis.
Figure 4 shows CBD Flux representative of deep permeability to the systemic circulatory fraction underlying the skin barrier in µg/cm²/hr for Test Articles A through F, at 0h-2h, 2h-4h, 4h-10h, 10h-24h, and 24h-48h.

### DETAILED DESCRIPTION

The presently disclosed subject matter now will be described more fully hereinafter. Like numbers refer to like elements throughout. The presently disclosed subject matter may be embodied in many different forms and should not be construed as limited to the embodiments set forth herein; rather, these embodiments are provided so that this disclosure will satisfy applicable legal requirements. Indeed, many modifications and other embodiments of the presently disclosed subject matter set forth herein will come to mind to one skilled in the art to which the presently disclosed subject matter pertains having the benefit of the teachings presented in the foregoing descriptions. Therefore, it is to be understood that the presently disclosed subject matter is not to be limited to the specific embodiments disclosed.

In some embodiments, the compositions or methods comprise the specified components or steps. In some embodiments, the compositions or methods consist of the specified components or steps. In other embodiments, the compositions or methods consist essentially of the specified components or steps. As used herein, "consists essentially of" the specified components or steps means that the composition includes at least the specified components or steps, and may also include other components or steps that do not materially affect the basic and novel characteristics of the invention.

The presently disclosed compositions and methods demonstrate efficacy for transdermal and/or dermal administration of active agents without the need for added penetration enhancers. However, although the presently disclosed compositions and methods do not require added penetration enhancers, such added penetration enhancers may be optionally incorporated.

### I. COMPOSITIONS

In one aspect, a transdermal and/or dermal composition comprising a lipophilic active agent is provided, wherein the transdermal and/or dermal composition comprises a lipophilic active agent in an oil-in-water emulsion comprising an aqueous phase and an oil phase, wherein the composition is obtainable by providing an oil phase comprising a dehydrated mixture, and wherein the dehydrated mixture comprises a therapeutically effective amount of the lipophilic active agent and an edible oil comprising long chain fatty acids and/or medium chain fatty acids, wherein the lipophilic active agent is cannabidiol and the edible oil is sunflower oil, and wherein the dehydrated mixture is obtainable by the steps of:i) combining the therapeutically effective amount of the cannabidiol with the sunflower oil; andii) dehydrating the product of step (i), thereby producing the dehydrated mixture; and combining the oil phase with an aqueous phase to provide the oil-in-water emulsion.A transdermal and/or dermal composition according to the invention comprises a lipophilic active agent in an oil-in-water emulsion comprising an aqueous phase and an oil phase, wherein the composition is obtainable by providing an oil phase comprising a dehydrated mixture, and wherein the dehydrated mixture comprises a therapeutically effective amount of the lipophilic active agent and an edible oil comprising long chain fatty acids and/or medium chain fatty acids, wherein the lipophilic active agent is cannabidiol and the edible oil is sunflower oil, and wherein the dehydrated mixture is obtainable by the steps of:i) combining the therapeutically effective amount of the cannabidiol with the sunflower oil; andii) dehydrating the product of step (i), thereby producing the dehydrated mixture; and combining the oil phase with an aqueous phase to provide the oil-in-water emulsion.

In other aspects, the bioavailability of the lipophilic active agent in a subject is at least 2 times greater than the bioavailability of the lipophilic active agent in the subject in the absence of the edible oil comprising long chain fatty acids and/or medium chain fatty acids, particularly at least 5 times greater, and more particularly at least 10 times greater. In another aspect, the edible oil comprising long chain fatty acids and/or medium chain fatty acids is substantially free of omega-6 fatty acids. In another aspect, the lipophilic active agent is selected from the group of cannabinoids, namely cannabidiol.

Specific components of the compositions are described in detail below.

### A. Cannabinoids

*Cannabis sativa* L. is one of the most widely used plants for both recreational and medicinal purposes. Over 500 natural constituents have been isolated and identified from *C*. *sativa* covering several chemical classes (Ahmed et al. (2008) J. Nat. Prod. 71:536-542; Ahmed et al. (2008) Tetrahedron Lett. 49:6050-6053; ElSohly & Slade (2005) Life Sci. 78:539-548; Radwan et al. (2009) J. Nat. Prod. 72:906-911; Radwan et al. (2008) Planta Medica. 74:267-272; Radwan et al. (2008) J. Nat. Prod. 69:2627-2633; Ross et al. (1995) Zagazig J. Pharm. Sci. 4:1-10; Turner et al. (1980) J. Nat. Prod. 43:169-170). Cannabinoids belong to the chemical class of terpenophenolics, of which at least 85 have been uniquely identified in cannabis (Borgelt et al. (2013) Pharmacotherapy 33:195-209).

Cannabinoids are ligands to cannabinoid receptors (CB₁, CB₂) found in the human body (Pertwee (1997) Pharmacol. Ther. 74:129-180). The cannabinoids are usually divided into the following groups: classical cannabinoids; non-classical cannabinoids; aminoalkylindole-derivatives; and eicosanoids (Pertwee (1997) Pharmacol. Ther. 74:129-180). Classical cannabinoids are those that have been isolated from *C*. *sativa* L. or their synthetic analogs. Non-classical cannabinoids are bi- or tri-cyclic analogs of tetrahydrocannabinol (THC) (without the pyran ring). Aminoalkylindoles and eicosanoids are substantially different in structure compared to classical and non-classical cannabinoids. The most common natural plant cannabinoids (phytocannabinoids) are cannabidiol (CBD), cannabigerol (CBG), cannabichromene (CBC), and cannabinol (CBN). The most psychoactive cannabinoid is Δ⁹-THC.

In recent years, marijuana and its components have been reported in scientific literature to counter the symptoms of a broad range of conditions including but not limited to multiple sclerosis and other forms of muscular spasm; movement disorders; pain, including migraine headache; glaucoma; asthma; inflammation; insomnia; and high blood pressure. There may also be utility for cannabinoids as anxiolytics, anti-convulsives, anti-depressants, anti-psychotics, anticancer agents, as well as appetite stimulants. Pharmacological and toxicological studies of cannabinoids have largely been focused on a synthetic analog of Δ⁹-THC (commercially available under the generic name Dronabinol). In 1985, Dronabinol was approved by the FDA for the treatment of chemotherapy associated nausea and vomiting, and later for AIDS-associated wasting and anorexia.

Therapeutic use of cannabinoids has been hampered by the psychoactive properties of some compounds (e.g., Dronabinol) as well as their low bioavailability when administered orally. Bioavailability refers to the extent and rate at which the active moiety (drug or metabolite) enters systemic circulation, thereby accessing the site of action. The low bioavailability of orally ingested cannabinoids (from about 6% to 20%; Adams & Martin (1996) Addiction 91: 1585-614; Agurell et al. (1986) Pharmacol. Rev. 38: 21-43; Grotenhermen (2003) Clin. Pharmacokinet. 42: 327-60) has been attributed to their poor dissolution properties and extensive first pass metabolism.

Cannabinoids are a heteromorphic group of chemicals which directly or indirectly activate the body's cannabinoid receptors. There are three main types of cannabinoids: herbal cannabinoids that occur uniquely in the cannabis plant, synthetic cannabinoids that are manufactured, and endogenous cannabinoids that are produced *in vivo.* Herbal cannabinoids are nearly insoluble in water but soluble in lipids, alcohol, and non-polar organic solvents. These natural cannabinoids are concentrated in a viscous resin that is produced in glandular structures known as trichomes. In addition to cannabinoids, the resin is rich in terpenes, which are largely responsible for the odor of the cannabis plant.

The identification of Δ⁹-tetrahydrocannabinol (THC) as a major psychoactive drug and its chemical synthesis in 1964 opened a new era of synthetic cannabinoids as pharmacological agents. Cannabinoid research has increased tremendously in recent years since the discovery of cannabinoid receptors and the endogenous ligands for these receptors. The receptors include CB1, predominantly expressed in the brain, and CB2, primarily found on the cells of the immune system. Cannabinoid receptors belong to a superfamily of G-protein-coupled receptors. They are single polypeptides with seven transmembrane α-helices, and have an extracellular, glycosylated N-terminus and intracellular C-terminus. Both CB1 and CB2 cannabinoid receptors are linked to G1/0-proteins. In addition to these receptors, endogenous ligands for these receptors capable of mimicking the pharmacological actions of THC have also been discovered. Such ligands were designated endocannabinoids and included anandamide and 2-arachidonoyl glycerol (2-AG). Anandamide is produced in the brain and peripheral immune tissues such as the spleen.

Unlike THC, which exerts its action by binding to CB1 and CB2, cannabidiol does not bind to these receptors and hence has no psychotropic activity. Instead, cannabidiol indirectly stimulates endogenous cannabinoid signaling by suppressing the enzyme that breaks down anandamide (fatty acid amide hydroxylase, "FAAH"). Cannabidiol also stimulates the release of 2-AG. Cannabidiol has been reported to have immunomodulating and anti-inflammatory properties, to exhibit anticonvulsive, anti-anxiety, and antipsychotic activity, and to function as an efficient neuroprotective antioxidant.

Cannabinoids in cannabis are often inhaled via smoking, but may also be ingested. Smoked or inhaled cannabinoids have reported bioavailabilities ranging from 2-56%, with an average of about 30% (Huestis (2007) Chem. Biodivers. 4:1770-1804; McGilveray (2005) Pain Res. Manag. 10 Suppl. A: 15A - 22A). This variability is mainly due to differences in smoking dynamics. Cannabinoids that are absorbed through the mucous membranes in the mouth (buccomucosal application) have bioavailabilities of around 13% (Karschner et al. (2011) Clin. Chem. 57:66-75). By contrast, when cannabinoids are ingested, bioavailability is typically reduced to about 6% (Karschner et al. (2011) Clin. Chem. 57:66-75).

Accordingly, within the compositions of the present invention, the lipophilic active agent is a cannabinoid.

In particular the cannabinoid within the compositions of the present invention is
CBD Cannabidiol

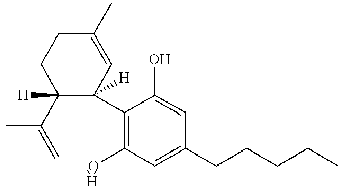

In particular aspects, at least one cannabinoid within the compositions of the present invention is cannabidiol, a non-psychoactive cannabinoid.

### B. Antioxidants

Antioxidants are chemicals that inhibit lipid oxidation. Some antioxidants (e.g., phenolic compounds) interrupt the free-radical chain of oxidative reactions by complexing with free radicals to form stable compounds that do not initiate or propagate further oxidation. Other antioxidants (e.g., acid compounds) slow the oxidative process by scavenging the reactive oxygen species. And still other antioxidants (e.g., chelators) slow oxidation by complexing with pro-oxidative metal ions.

Thousands of different types of antioxidants exist in nature. Some antioxidants of most importance to human health include without limitation astaxanthin, enzymes such as Superoxide Dismusase, vitamins A, C, and E, beta-carotene, selenium, lycopene, lutein, Coenzyme Q10, phytic acid, flavonoids, and polyphenols. Antioxidants are also separated into categories based upon whether they are water-soluble (hydrophilic) or fat-soluble (hydrophobic or lipophilic). Water-soluble antioxidants tend to predominantly react with oxidants in the cell cytosol and the blood plasma, while fat-soluble antioxidants tend to protect cell membranes from lipid peroxidation.

Various antioxidant compositions have been developed for the stabilization of oils and fats; most are mixtures of natural phenolic compounds (e.g., tocopherols) and acid compounds (e.g., ascorbic acid). While these antioxidant compositions inhibit lipid oxidation, they are not nearly as effective as synthetic phenolic antioxidants. One of the most effective antioxidants is ethoxyquin (6-ethoxy-1,2-dihydro-2,2,4-trimethylquinoline, sold under the trademark SANTOQUIN^{®}), which is widely used as an antioxidant or preservative in feed supplements and a variety of other applications.

Several antioxidants are suitable for use in the compositions.

The antioxidant may be a compound that interrupts the free-radical chain of oxidative reactions by protonating free radicals, thereby inactivating them. The antioxidant may be a compound that scavenges the reactive oxygen species. Alternatively, the antioxidant may be a compound that chelates the metal catalysts. The antioxidant may be a synthetic compound, a semisynthetic compound, or a natural (or naturally-derived) compound.

In some aspects, the antioxidant is a substituted 1,2-dihydroquinoline. Substituted 1,2-dihydroquinoline compounds suitable for use generally correspond to Formula (I) as described in U.S. Patent App. Pub. No. US20080019860, particularly where the substituted 1,2-dihydroquinoline is 6-ethoxy-1,2-dihydro-2,2,4-trimethylquinoline (commonly known as ethoxyquin and sold under the trademark SANTOQUIN^{®}) having the structure:

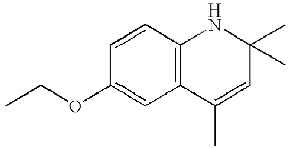

In other aspects, the antioxidant includes, but is not limited to, ascorbic acid and its salts, ascorbyl palmitate, ascorbyl stearate, anoxomer, N-acetylcysteine, benzyl isothiocyanate, o-, m- or p-amino benzoic acid (o is anthranilic acid, p is PABA), butylated hydroxyanisole (BHA), butylated hydroxytoluene (BHT), caffeic acid, canthaxantin, alpha-carotene, beta-carotene, beta-caraotene, beta-apo-carotenoic acid, carnosol, carvacrol, catechins, cetyl gallate, chlorogenic acid, citric acid and its salts, clove extract, coffee bean extract, p-coumaric acid, 3,4-dihydroxybenzoic acid, N,N'-diphenyl-p-phenylenediamine (DPPD), dilauryl thiodipropionate, distearyl thiodipropionate, 2,6-di-tert-butylphenol, dodecyl gallate, edetic acid, ellagic acid, erythorbic acid, sodium erythorbate, esculetin, esculin, 6-ethoxy-1,2-dihydro-2,2,4-trimethylquinoline, ethyl gallate, ethyl maltol, ethylenediaminetetraacetic acid (EDTA), eucalyptus extract, eugenol, ferulic acid, flavonoids, flavones (e.g., apigenin, chrysin, luteolin), flavonols (e.g., datiscetin, myricetin, daemfero), flavanones, fraxetin, fumaric acid, gallic acid, gentian extract, gluconic acid, glycine, gum guaiacum, hesperetin, alpha-hydroxybenzyl phosphinic acid, hydroxycinammic acid, hydroxyglutaric acid, hydroquinone, N-hydroxysuccinic acid, hydroxytryrosol, hydroxyurea, ice bran extract, lactic acid and its salts, lecithin, lecithin citrate; R-alpha-lipoic acid, lutein, lycopene, malic acid, maltol, 5-methoxy tryptamine, methyl gallate, monoglyceride citrate; monoisopropyl citrate; morin, beta-naphthoflavone, nordihydroguaiaretic acid (NDGA), octyl gallate, oxalic acid, palmityl citrate, phenothiazine, phosphatidylcholine, phosphoric acid, phosphates, phytic acid, phytylubichromel, pimento extract, propyl gallate, polyphosphates, quercetin, trans-resveratrol, rosemary extract, rosmarinic acid, sage extract, sesamol, silymarin, sinapic acid, succinic acid, stearyl citrate, syringic acid, tartaric acid, thymol, tocopherols (i.e., alpha-, beta-, gamma- and delta-tocopherol), tocotrienols (i.e., alpha-, beta-, gamma- and delta-tocotrienols), tyrosol, vanilic acid, 2,6-di-tert-butyl-4-hydroxymethylphenol (i.e., lonox 100), 2,4-(tns-3',5'-bi-tert-butyl-4'-hydroxybenzyl)-mesitylene (i.e., lonox 330), 2,4,5-trihydroxybutyrophenone, ubiquinone, tertiary butyl hydroquinone (TBHQ), thiodipropionic acid, trihydroxy butyrophenone, tryptamine, tyramine, uric acid, vitamin K and derivates, vitamin Q10, wheat germ oil, zeaxanthin, or combinations thereof.

Further exemplary antioxidants include synthetic phenolic compounds, such as tertiary butyl hydroquinone (TBHQ); gallic acid derivatives, such as n-propyl gallate; vitamin C derivatives, such as ascorbyl palmitate; lecithin; and vitamin E compounds, such as, alpha-tocopherol .

### C. Bioavailability Enhancing Agents

Bioavailability refers to the extent and rate at which the active moiety (drug or metabolite) enters systemic circulation, thereby accessing the site of action. Bioavailability for a given formulation provides an estimate of the relative fraction of the orally administered dose that is absorbed into the systemic circulation. Low bioavailability is most common with oral dosage forms of poorly water-soluble, slowly absorbed drugs. Insufficient time for absorption in the gastrointestinal tract is a common cause of low bioavailability. If the drug does not dissolve readily or cannot penetrate the epithelial membrane (e.g., if it is highly ionized and polar), time at the absorption site may be insufficient. Orally administered drugs must pass through the intestinal wall and then the portal circulation to the liver, both of which are common sites of first-pass metabolism (metabolism that occurs before a drug reaches systemic circulation). Thus, many drugs may be metabolized before adequate plasma concentrations are reached.

Bioavailability is usually assessed by determining the area under the plasma concentration-time curve (AUC). AUC is directly proportional to the total amount of unchanged drug that reaches systemic circulation. Plasma drug concentration increases with extent of absorption; the maximum (peak) plasma concentration is reached when drug elimination rate equals absorption rate. Peak time is the most widely used general index of absorption rate; the slower the absorption, the later the peak time.

The bioavailability of some drugs is increased when co-administered with food, particularly agents such as cannabinoids that are Class II drugs under the Biopharmaceutical Drug Classification System (Kelepu et al. (2013) Acta Pharmaceutica Sinica B 3:361-372; Amidon et al. (1995) Pharm. Res. 12:413-420; Charman et al. (1997) J. Pharm. Sci. 86:269-282; Winstanley et al. (1989) Br. J. Clin. Pharmacol. 28:621-628). It is the lipid component of the food that plays a key role in the absorption of lipophilic drugs and that leads to enhanced oral bioavailability (Hunt & Knox (1968) J. Physiol. 194:327-336; Kelepu et al. (2013) Acta Pharmaceutica Sinica B 3:361-372). This has been attributed to the ability of a high fat meal to stimulate biliary and pancreatic secretions, to decrease metabolism and efflux activity, to increase intestinal wall permeability, and to a prolongation of gastrointestinal tract (GIT) residence time and transport via the lymphatic system (Wagnera et al. (2001) Adv. Drug Del. Rev. 50:S13-31; Kelepu et al. (2013) Acta Pharmaceutica Sinica B 3:361-372). High fat meals also elevate triglyceride-rich lipoproteins that associate with drug molecules and enhance intestinal lymphatic transport, which leads to changes in drug disposition and changes the kinetics of the pharmacological actions of poorly soluble drugs (Gershkovich et al. (2007) Eur. J. Pharm. Sci. 32:24-32; Kelepu et al. (2013) Acta Pharmaceutica Sinica B 3:361-372). However, coadministration of food with lipophilic drugs requires close control and/or monitoring of food intake when dosing such drugs, and can also be subject to problems with patient compliance (Kelepu et al. (2013) Acta Pharmaceutica Sinica B 3:361-372).

In some aspects, within the compositions of the present invention, the bioavailability enhancing agent is an edible oil or fat comprising medium and/or long chain fatty acids. An edible oil is defined herein as an oil that is capable of undergoing de-esterification or hydrolysis in the presence of pancreatic lipase *in vivo* under normal physiological conditions. Specifically, digestible oils may be complete glycerol triesters of medium chain (C₇-C₁₃) or long chain (C₁₄-C₂₂) fatty acids with low molecular weight (up to C₆) mono-, di- or polyhydric alcohols. Medium and long chain fatty acids can comprise oleic acid, undecanoic acid, valeric acid, heptanoic acid, pelargonic acid, capric acid, lauric acid, and eicosapentaenoic acid.

Some examples of edible oils (also referred to as digestible oils) include: vegetable, nut, or seed oils (such as coconut oil, peanut oil, soybean oil, safflower seed oil, corn oil, olive oil, castor oil, cottonseed oil, arachis oil, sunflower seed oil, coconut oil, palm oil, rapeseed oil, evening primrose oil, grape seed oil, wheat germ oil, sesame oil, avocado oil, almond, borage, peppermint and apricot kernel oils), and animal oils (such as fish liver oil, shark oil and mink oil). The compositions according to the invention comprise sunflower oil.

In a further aspect, the bioavailability enhancing agent is substantially free of omega-6 fatty acids.

In other aspects, the bioavailability of the lipophilic active agent in a subject is at least about 1.5 times, 2 times, 2.5 times, 3 times, 3.5 times, 4 times, 4.5 times, 5 times, 5.5 times, 6 times, 6.5 times, 7 times, 7.5 times, 8 times, 8.5 times, 9 times, 9.5 times, 10 times greater, 10.5 times greater, 11 times greater, 11.5 times greater, 12 times greater, 12.5 times greater, 13 times greater, 13.5 times greater, 14 times greater, 14.5 times greater, 15 times greater, 15.5 times greater, 16 times greater, 16.5 times greater, 17 times greater, 17.5 times greater, 18 times greater, 18.5 times greater, 19 times greater, 19.5 times greater, or 20 times greater than the bioavailability of the lipophilic active agent in the subject in the absence of the bioavailability enhancing agent.

In a further aspect, the bioavailability of the lipophilic active agent in a subject is greater than 20% or at least about 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, or greater.

Assays and methods for measuring lipophilic active agent bioavailability are well known in the art (see, *e.g*., Rocci & Jusko (1983) Comput. Programs Biomed. 16:203-215; Shargel & Yu (1999) Applied biopharmaceutics & pharmacokinetics (4th ed.). New York: McGraw-Hill; Hu & Li (2011) Oral Bioavailability: Basic Principles, Advanced Concepts, and Applications, John Wiley & Sons Ltd.; Karschner et al. (2011) Clinical Chemistry 57:66-75; Ohlsson et al. (1980) Clin. Pharmacol. Ther. 28:409-416; Ohlsson et al. (1982) Biomed. Environ. Mass Spectrom. 9:6-10; Ohlsson et al. (1986) Biomed. Environ. Mass Spectrom. 13:77-83; Karschner et al. (2010) Anal. Bioanal. Chem. 397:603-611).

### D. Dosages and Concentrations

The active agents of the present invention are effective over a wide dosage range. For example, in treating adult humans, compositions may comprise dosages of lipophilic active agents from 0.01 mg to 1,000 mg, from 0.5 mg to 500 mg, from 1 mg to 100 mg, from 5 mg to 50 mg, and from 10 mg to 25 mg. Alternatively, in treating adult humans, compositions may comprise dosages of lipophilic active agents of 0.01 mg, 0.05 mg, 0.1 mg, 0.25 mg, 0.5 mg, 0.75 mg, 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, or 1,000 mg.

In other aspects, the concentration of lipophilic active agents within the compositions may range from 5 ppm to about 1000 ppm. In other embodiments, the concentration may range from about 50 to about 500 ppm. In still additional embodiments, the concentration may range from about 50 to about 200 ppm, particularly about 100 ppm.

In other aspects, the concentration of lipophilic active agents within the compositions and methods of the present invention may also be expressed as a percent of the active agent by weight. In one embodiment, the amount of the lipophilic active agent may range from about 0.0001% to about 20% by weight. In another embodiment, the amount may range from about of 1% to about 15% by weight. In yet another embodiment, the amount may range from 3,75% to about 10% by weight. In another embodiment, the amount may range from about 1% to about 99% by weight, from about 10% to about 80% by weight, and more typically, from about 20% to about 60% by weight. In another embodiment, the amount may be about 5% by weight, less than about 5% by weight, less than about 4% by weight, less than about 3% by weight, less than about 2% by weight, or less than about 1% by weight, In another embodiment, the amount may be greater than about 5%, greater than about 10%, greater than about 15%, greater than about 20%, greater than about 25%, greater than about 30%, greater than about 35%, greater than about 40%, greater than about 50%, greater than about 55%, greater than about 60%, greater than about 65%, greater than about 70%), or greater than about 75%.

In other aspects, the concentration of lipophilic active agents will vary depending on the total number of lipophilic active agents. For example, in compositions comprising more than one lipophilic active agent, the concentration of each lipophilic active may range from about 100 ppm to about 400 ppm (or from about 3,75% to about 30% by weight), with the total concentration of lipophilic active agents ranging from about 50 ppm to about 2000 ppm.

It is contemplated that the compositions of the present invention can include any ingredient (e.g., lipophilic active agent, carrier, etc.) or any combination thereof described throughout this specification. The concentrations of the any ingredient within the compositions can vary. In non-limiting embodiments, for example, the compositions can comprise, consisting essentially of, or consist of, in their final form, for example, at least about 0.0001%, 0.0002%, 0.0003%, 0.0004%, 0.0005%, 0.0006%, 0.0007%, 0.0008%, 0.0009%, 0.0010%, 0.0011%, 0.0012%, 0.0013%, 0.0014%, 0.0015%, 0.0016%, 0.0017%, 0.0018%, 0.0019%, 0.0020%, 0.0021%, 0.0022%, 0.0023%, 0.0024%, 0.0025%, 0.0026%, 0.0027%, 0.0028%, 0.0029%, 0.0030%, 0.0031%, 0.0032%, 0.0033%, 0.0034%, 0.0035%, 0.0036%, 0.0037%, 0.0038%, 0.0039%, 0.0040%, 0.0041%, 0.0042%, 0.0043%, 0.0044%, 0.0045%, 0.0046%, 0.0047%, 0.0048%, 0.0049%, 0.0050%, 0.0051%, 0.0052%, 0.0053%, 0.0054%, 0.0055%, 0.0056%, 0.0057%, 0.0058%, 0.0059%, 0.0060%, 0.0061%, 0.0062%, 0.0063%, 0.0064%, 0.0065%, 0.0066%, 0.0067%, 0.0068%, 0.0069%, 0.0070%, 0.0071%, 0.0072%, 0.0073%, 0.0074%, 0.0075%, 0.0076%, 0.0077%, 0.0078%, 0.0079%, 0.0080%, 0.0081%, 0.0082%, 0.0083%, 0.0084%, 0.0085%, 0.0086%, 0.0087%, 0.0088%, 0.0089%, 0.0090%, 0.0091%, 0.0092%, 0.0093%, 0.0094%, 0.0095%, 0.0096%, 0.0097%, 0.0098%, 0.0099%, 0.0100%, 0.0200%, 0.0250%, 0.0275%, 0.0300%, 0.0325%, 0.0350%, 0.0375%, 0.0400%, 0.0425%, 0.0450%, 0.0475%, 0.0500%, 0.0525%, 0.0550%, 0.0575%, 0.0600%, 0.0625%, 0.0650%, 0.0675%, 0.0700%, 0.0725%, 0.0750%, 0.0775%, 0.0800%, 0.0825%, 0.0850%, 0.0875%, 0.0900%, 0.0925%, 0.0950%, 0.0975%, 0.1000%, 0.1250%, 0.1500%, 0.1750%, 0.2000%, 0.2250%, 0.2500%, 0.2750%, 0.3000%, 0.3250%, 0.3500%, 0.3750%, 0.4000%, 0.4250%, 0.4500%, 0.4750%, 0.5000%, 0.5250%, 0.0550%, 0.5750%, 0.6000%, 0.6250%, 0.6500%, 0.6750%, 0.7000%, 0.7250%, 0.7500%, 0.7750%, 0.8000%, 0.8250%, 0.8500%, 0.8750%, 0.9000%, 0.9250%, 0.9500%, 0.9750%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, 5.1%, 5.2%, 5.3%, 5.4%, 5.5%, 5.6%, 5.7%, 5.8%, 5.9%, 6.0%, 6.1%, 6.2%, 6.3%, 6.4%, 6.5%, 6.6%, 6.7%, 6.8%, 6.9%, 7.0%, 7.1%, 7.2%, 7.3%, 7.4%, 7.5%, 7.6%, 7.7%, 7.8%, 7.9%, 8.0%, 8.1%, 8.2%, 8.3%, 8.4%, 8.5%, 8.6%, 8.7%, 8.8%, 8.9%, 9.0%, 9.1%, 9.2%, 9.3%, 9.4%, 9.5%, 9.6%, 9.7%, 9.8%, 9.9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 60%, 65%, 70%, 75%, 80%, 85%, 90%, 95%, or 99% or any range derivable therein, of at least one of the ingredients that are mentioned throughout the specification and claims. In non-limiting aspects, the percentage can be calculated by weight or volume of the total composition. A person of ordinary skill in the art would understand that the concentrations can vary depending on the addition, substitution, and/or subtraction of ingredients in a given composition.

### E. Additional Transdermal and/or Dermal Formulation Components

The compositions can be incorporated into all types of vehicles. Non-limiting examples of suitable vehicles include emulsions (e.g., water-in-oil, water-in-oil-in-water, oil-in-water, silicone-in-water, water-in-silicone, oil-in-water-in-oil, oil-in-water-in-silicone emulsions), creams, lotions, solutions (both aqueous and hydro-alcoholic), anhydrous bases (such as lipsticks and powders), gels, and ointments or by other method or any combination of the forgoing as would be known to one of ordinary skill in the art. Variations and other appropriate vehicles will be apparent to the skilled artisan and are appropriate for use.

In certain aspects, it is important that the concentrations and combinations of the compounds, ingredients, and agents be selected in such a way that the combinations are chemically compatible and do not form complexes which precipitate from the finished product.

It is also contemplated that ingredients identified throughout this specification can be individually or combinatorially encapsulated for delivery to a target area such as skin. Non-limiting examples of encapsulation techniques include the use of liposomes, vesicles, and/or nanoparticles (e.g., biodegradable and non-biodegradable colloidal particles comprising polymeric materials in which the ingredient is trapped, encapsulated, and/or absorbed--examples include nanospheres and nanocapsules) that can be used as delivery vehicles to deliver the ingredient to skin (see, e.g., U.S. Patent Nos. 6,387,398; 6,203,802; and 5,411,744).

The composition can also be used in many cosmetic products including, but not limited to, sunscreen products, sunless skin tanning products, hair products, finger nail products, moisturizing creams, skin benefit creams and lotions, softeners, day lotions, gels, ointments, foundations, night creams, lipsticks, cleansers, toners, masks, or other known cosmetic products or applications. Additionally, the cosmetic products can be formulated as leave-on or rinse-off products. In certain aspects, the compositions can be stand-alone products.

The Personal Care Product Council's International Cosmetic Ingredient Dictionary and Handbook, Thirteenth Edition, and the CTFA Cosmetic Ingredient Handbook, Second Edition (1992) each describe a wide variety of non-limiting cosmetic and pharmaceutical ingredients commonly used in the skin care industry, which are suitable optional components for use in the compositions.

Examples of these ingredient classes include: abrasives, absorbents, aesthetic components such as fragrances, pigments, colorings/colorants, plant extracts including essential oils, anti-caking agents, antifoaming agents, antimicrobials, binders, biological additives, buffering agents, bulking agents, chelating agents, colorants, cosmetic astringents, cosmetic biocides, denaturants, drug astringents, emollients, external analgesics, film formers or materials, opacifying agents, pH adjusters, preservatives, propellants, reducing agents, sequestrants, skin cooling agents, skin protectants, thickeners/viscosity modifiers, vitamins, and combinations thereof.

The compositions disclosed herein may also include one or more pharmaceutically acceptable excipients. As utilized herein, the term "excipient" generally refers to any substance, not itself a bioactive agent, used in conjunction with the bioactive agent(s) delivered to a subject to improve one of more characteristics, such as its handling or storage properties or to permit or facilitate formation of a dose unit of the composition. Excipients include, by way of illustration and not limitation, solvents (e.g., lower alcohol, such as ethanol or isopropanol; or water), penetration enhancers, thickening agents, wetting agents, lubricants, emollients, essential oils, substances added to mask or counteract a disagreeable odor or flavor, fragrances, adjuvants, and substances added to improve appearance or texture of the composition or delivery device. Any such excipients can be used in any amounts as are generally known.

### i. Penetration Enhancers

The disclosed compositions may also include various antioxidants to retard oxidation of one or more components. Additionally, the prevention of the action of microorganisms can be brought about by preservatives such as various antibacterial and antifungal agents, including but not limited to parabens (e.g., methylparabens, propylparabens), chlorobutanol, phenol, sorbic acid, thimerosal or combinations thereof. In some embodiments, the compositions do not contain parabens.

An important feature of the compositions disclosed herein is that the formulations exhibit suitable skin penetration to achieve the required therapeutic objective and advantageous organoleptic properties, such as suitable consistency without adherence (sticking) to the skin or clothing. There is furthermore a need for the formulation to have good physical-chemical stability, especially in the cold, and microbiological stability. In case of low-solubility active ingredients (e.g., celecoxib), the formulation should also improve the solubility of the active ingredient. Moreover, said formulations should be easily manufacturable on an industrial scale.

Transdermal pharmaceutical formulations are characterized by the measurement of the skin penetration of the active ingredient. Such a measurement method and apparatus developed are disclosed in US Patent App. Pub. No. 20120024743.

The presently disclosed compositions and methods demonstrate efficacy for transdermal and/or dermal administration of active agents without the need for added penetration enhancers. However, although the presently disclosed compositions and methods do not require added penetration enhancers, such added penetration enhancers may be optionally incorporated.

Non-limiting examples of penetration enhancing agents include C₈-C₂₂ fatty acids such as isostearic acid, octanoic acid, and oleic acid; C₈-C₂₂ fatty alcohols such as oleyl alcohol and lauryl alcohol; lower alkyl esters of C₈-C₂₂ fatty acids such as ethyl oleate, isopropyl myristate, butyl stearate, and methyl laurate; di(lower)alkyl esters of C₆-C₂₂ diacids such as diisopropyl adipate; monoglycerides of C₈-C₂₂ fatty acids such as glyceryl monolaurate; tetrahydrofurfuryl alcohol polyethylene glycol ether; polyethylene glycol, propylene glycol; 2-(2-ethoxyethoxy)ethanol; diethylene glycol monomethyl ether; alkylaryl ethers of polyethylene oxide; polyethylene oxide monomethyl ethers; polyethylene oxide dimethyl ethers; dimethyl sulfoxide; glycerol; ethyl acetate; acetoacetic ester; and N-alkylpyrrolidone.

Penetration enhancers may also include terpenes such as isoborneol, irone, ocimene, carveol, carvotanacetone, carvomenthone, carvone, carene, carone, camphene, camphor, geraniol, cymene, sabinene, safranal, cyclocitral, citral, citronellal, citronellic acid, citronellol, cineole, sylvestrene, thujyl alcohol, thujone, terpineol, terpinene, terpinolene, tricyclene, nerol, pinene, pinocampheol, pinol, piperitenone, phellandral, phellandrene, fenchene, fenchyl alcohol, perillyl alcohol, perillyl aldehyde, borneol, myrcene, menthol, menthone, ionol, ionone, linalool, or limonene.

One or more penetration enhancers, when present, can generally be present in a total amount of from about 0.01% to about 25%, or from about 0.1% to about 15% by weight of the composition.

### ii. Essential Oils

Essential oils include oils derived from herbs, flowers, trees, and other plants. Such oils are typically present as tiny droplets between the plant's cells, and can be extracted by several methods known to those of skill in the art (e.g., steam distilled, enfleurage (i.e., extraction by using fat), maceration, solvent extraction, or mechanical pressing). When these types of oils are exposed to air they tend to evaporate (i.e., a volatile oil). As a result, many essential oils are colorless, but with age they can oxidize and become darker. Essential oils are insoluble in water and are soluble in alcohol, ether, fixed oils (vegetal), and other organic solvents. Typical physical characteristics found in essential oils include boiling points that vary from about 160° to 240° C. and densities ranging from about 0.759 to about 1.096.

Essential oils typically are named by the plant from which the oil is found. For example, rose oil or peppermint oil are derived from rose or peppermint plants, respectively. Non-limiting examples of essential oils that can be used include sesame oil, macadamia nut oil, tea tree oil, evening primrose oil, Spanish sage oil, Spanish rosemary oil, coriander oil, thyme oil, pimento berries oil, rose oil, anise oil, balsam oil, bergamot oil, rosewood oil, cedar oil, chamomile oil, sage oil, clary sage oil, clove oil, cypress oil, eucalyptus oil, fennel oil, sea fennel oil, frankincense oil, geranium oil, ginger oil, grapefruit oil, jasmine oil, juniper oil, lavender oil, lemon oil, lemongrass oil, lime oil, mandarin oil, marjoram oil, myrrh oil, neroli oil, orange oil, patchouli oil, pepper oil, black pepper oil, petitgrain oil, pine oil, rose otto oil, rosemary oil, sandalwood oil, spearmint oil, spikenard oil, vetiver oil, wintergreen oil, or ylang ylang. Other essential oils known to those of skill in the art are also contemplated as being useful.

### iii. Plant Extracts

In one embodiment, the composition comprises one or more plant extracts that are not essential oils. The plant extract may be one or more extracts obtained from plant leaves, stems, petals, seeds, roots and/or pollen, for example one or more extracts obtained from plant leaves, stems, and/or roots. In one embodiment, the plant extract is obtained from a plant selected from: aloe vera, basil, birch, burdock, comfrey, chamomile (including German chamomile), calendula, dandelion, echinacea, elderflower, green tea, fennel, horsetail, hyssop, lady's mantle, lavender, lemon balm, lime flower, linden, liquorice, marshmallow, nettle, Oregon grape, plantain, pomegranate, rose, rosemary, sage, St. John's wort, yarrow and witch hazel. The extract may be obtained from the plant's leaves, stems, petals, seeds, roots and/or pollen. For example, it may be obtained from the plant's leaves, stems, and/or roots.

### iv. Thickening Agents

Thickening agents, including thickener or gelling agents, include substances which that can increase or control the viscosity of a composition. Thickeners includes those that can increase the viscosity of a composition without substantially modifying the efficacy of the active ingredient within the composition. Thickeners can also increase the stability of the compositions. Thickeners include hydrogenated polyisobutene or trihydroxystearin, or a mixture of both.

Non-limiting examples of additional thickening agents that can be used include carboxylic acid polymers, crosslinked polyacrylate polymers, polyacrylamide polymers, polysaccharides, and gums. Examples of carboxylic acid polymers include crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol (see U.S. Pat. Nos. 5,087,445; 4,509,949; 2,798,053; CTFA International Cosmetic Ingredient Dictionary, Fourth edition, 1991, pp. 12 and 80). Examples of commercially available carboxylic acid polymers include carbomers, which are homopolymers of acrylic acid crosslinked with allyl ethers of sucrose or pentaerytritol (e.g., Carbopol.TM. 900 series from B. F. Goodrich).

Non-limiting examples of crosslinked polyacrylate polymers include cationic and nonionic polymers. Examples are described in U.S. Pat. Nos. 5,100,660; 4,849,484; 4,835,206; 4,628,078; 4,599,379.

Non-limiting examples of polyacrylamide polymers (including nonionic polyacrylamide polymers including substituted branched or unbranched polymers) include polyacrylamide, isoparaffin and laureth-7, multi-block copolymers of acrylamides and substituted acrylamides with acrylic acids and substituted acrylic acids.

Non-limiting examples of polysaccharides include cellulose, carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof. Another example is an alkyl substituted cellulose where the hydroxy groups of the cellulose polymer is hydroxyalkylated (preferably hydroxy ethylated or hydroxypropylated) to form a hydroxyalkylated cellulose which is then further modified with a C₁₀-C₃₀ straight chain or branched chain alkyl group through an ether linkage. Typically these polymers are ethers of C₁₀-C₃₀ straight or branched chain alcohols with hydroxyalkylcelluloses. Other useful polysaccharides include scleroglucans comprising a linear chain of (1-3) linked glucose units with a (1-6) linked glucose every three unit.

Non-limiting examples of gums that can be used include acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluroinic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboyxmethyl dextran, sodium carrageenan, tragacanth gum, xanthan gum, and mixtures thereof.

Further non-limiting examples of thickening agents include carbomer, cetyl alcohol, ammonium acryloydimethyltaurate/VP copolymer, aluminum starch actenylsuccinate, cocamidopropyl betaine, PPG-2 hydroxyethyl coco/isostearamide, tin oxide, hexadecane copolymer, calcium aluminum borosilicate, alumina, calcium sodium borosilicate, aluminum calcium sodium silicate, synthetic fluorphlogopite, dipropylene glycol, quaternium-90 bentonite, and disodium EDTA.

Thickening agents may also include anionic polymers such as polyacrylic acid (CARBOPOL^{™}), carboxypolymethylene, carboxymethylcellulose and the like, including derivatives of CARBOPOL^{™} polymers, such as CARBOPOL^{™} Ultrez 10, CARBOPOL^{™} 940, CARBOPOL^{™} 941, CARBOPOL^{™} 954, CARBOPOL^{™} 980, CARBOPOL^{™} 981, CARBOPOL^{™} ETD 2001, CARBOPOL^{™} EZ-2 and CARBOPOL^{™}EZ-3, and other polymers such as PEMULEN^{™} polymeric emulsifiers, and NOVEON^{™} polycarbophils. Thickening agents, when present, can generally be present in a total amount by weight of from about 0.1% to about 15%, from about 0.25% to about 10%, or from about 0.5% to about 5%.

### v. Neutralizing Agents

One or more neutralizing agents can be present to assist in forming a gel. Suitable neutralizing agents include sodium hydroxide (e.g., as an aqueous mixture), potassium hydroxide (*e*.*g*., as an aqueous mixture), ammonium hydroxide (*e.g*., as an aqueous mixture), triethanolamine, tromethamine (2-amino 2-hydroxymethyl-1,3 propanediol), aminomethyl propanol (AMP), tetrahydroxypropyl ethylene diamine, diisopropanolamine, Ethomeen C-25 (Armac Industrial Division), Di-2 (ethylhexyl) amine (BASF-Wyandotte Corp., Intermediate Chemicals Division), triamylamine, Jeffamine D-1000 (Jefferson Chemical Co.), b-Dimethylaminopropionitrite (American Cyanamid Co.), Armeen CD (Armac Industrial Division), Alamine 7D (Henkel Corporation), dodecylamine, and morpholine. The neutralizing agent can be present in an amount sufficient to form a gel which is suitable for contact with the skin of a mammal, e.g., up to about 10% by weight of the composition, for example between about 0.1% and about 5% by weight of the composition.

### vi. Wetting Agents (Surfactants)

Compositions of the present invention may also include one or more pharmaceutically acceptable wetting agents (also referred to as surfactants) as excipients. Non-limiting examples of surfactants can include quaternary ammonium compounds, for example benzalkonium chloride, benzethonium chloride and cetylpyridinium chloride, dioctyl sodium sulfosuccinate, polyoxyethylene alkylphenyl ethers, for example nonoxynol 9, nonoxynol 10, and octoxynol 9, poloxamers (polyoxyethylene and polyoxypropylene block copolymers), polyoxyethylene fatty acid glycerides and oils, for example polyoxyethylene (8) caprylic/capric mono- and diglycerides (*e.g*., LABRASOL^{™} of Gattefosse), polyoxyethylene (35) castor oil and polyoxyethylene (40) hydrogenated castor oil; polyoxyethylene alkyl ethers, for example polyoxyethylene (20) cetostearyl ether, polyoxyethylene fatty acid esters, for example polyoxyethylene (40) stearate, polyoxyethylene sorbitan esters, for example polysorbate 20 and polysorbate 80 (*e.g*., TWEEN^{™} 80 of ICI), propylene glycol fatty acid esters, for example propylene glycol laurate (*e.g*., LAUROGLYCOL^{™} of Gattefosse), sodium lauryl sulfate, fatty acids and salts thereof, for example oleic acid, sodium oleate and triethanolamine oleate, glyceryl fatty acid esters, for example glyceryl monostearate, sorbitan esters, for example sorbitan monolaurate, sorbitan monooleate, sorbitan monopalmitate and sorbitan monostearate, tyloxapol, and mixtures thereof. One or more wetting agents, when present, generally constitute in total from about 0.25% to about 15%, from about 0.4% to about 10%, or from about 0.5% to about 5%, of the total weight of the composition.

### vii. Lubricants

Compositions may also include one or more pharmaceutically acceptable lubricants (including anti-adherents and/or glidants) as excipients. Suitable lubricants include, without limiation, glyceryl behapate (*e.g*., COMPRITOL^{™} 888); stearic acid and salts thereof, including magnesium (magnesium stearate), calcium and sodium stearates; hydrogenated vegetable oils (*e.g*., STEROTEX^{™}); colloidal silica; talc; waxes; boric acid; sodium benzoate; sodium acetate; sodium fumarate; sodium chloride; DL-leucine; PEG (e.g., CARBOWAX^{™} 4000 and CARBOWAX^{™} 6000); sodium oleate; sodium lauryl sulfate; and magnesium lauryl sulfate. Such lubricants can generally constitute from about 0.1% to about 10%, from about 0.2% to about 8%, or from about 0.25% to about 5%, of the total weight of the composition.

### viii. Emollients

Compositions of the present invention may also include one or more emollients. Illustrative emollients include, without limitation, mineral oil, mixtures of mineral oil and lanolin alcohols, cetyl alcohol, cetostearyl alcohol, petrolatum, petrolatum and lanolin alcohols, cetyl esters wax, cholesterol, glycerin, glyceryl monostearate, isopropyl myristate, isopropyl palmitate, lecithin, allyl caproate, althea officinalis extract, arachidyl alcohol, argobase EUC, Butylene glycol dicaprylate/dicaprate, acacia, allantoin, carrageenan, cetyl dimethicone, cyclomethicone, diethyl succinate, dihydroabietyl behenate, dioctyl adipate, ethyl laurate, ethyl palmitate, ethyl stearate, isoamyl laurate, octanoate, PEG-75 lanolin, sorbitan laurate, walnut oil, wheat germ oil super refined almond, super refined sesame, super refined soybean, octyl palmitate, caprylic/capric triglyceride and glyceryl cocoate. A composition may include one or more emollients in a total amount of from about 1% to about 30%, from about 3% to about 25%, or from about 5% to about 15%, by weight of the composition.

### ix. Emulsifying Agents

Compositions of the present invention may also include one or more emulsifying agents. As utilized herein, the term "emulsifying agent" generally refers to an agent capable of lowering surface tension between a non-polar and polar phase and includes compounds defined as "self-emulsifying" agents. Suitable emulsifying agents can come from any class of pharmaceutically acceptable emulsifying agents including carbohydrates, proteins, high molecular weight alcohols, wetting agents, waxes and finely divided solids. One or more emulsifying agents, when present, can be present in a composition in a total amount of from about 1% to about 15%, from about 1% to about 12%, from about 1% to about 10%, or from about 1% to about 5% by weight of the composition.

### x. Solubilizers

Solubilizers are well known in the state of the art. Solubilizers can be preferably selected from polyethylene glycols, sorbitol esters with fatty acids, pegylated sorbitol esters with fatty acids (polysorbates), polyethylene glycol alkylethers, polyoxyethylene and polyoxypropylene block polymers and silicone alkyl glycols. The solubilizer polyethylene glycol hexadecyl ether or polyoxyethylene (20) cetyl ether is particularly suitable for the solubility enhancement of COX-2 inhibitor compounds, especially of celecoxib. In another aspect, an additional solubilizer includes poly(ethylene glycol)-block-poly(propylene glycol)-block-poly(ethylene glycol), which may also be used in combination with tween 60 and ethanol. Further advantageous combinations of solubilizers include but are not limited to combinations with polyethylene glycol 1000 (PEG 1000) and Tween 60 (polyoxyethylene (20) sorbitan monostearate).

### xi. Oils and Waxes

Combinations of one or more oils and/or one or more waxes may be used.

Liquid oils that can be mentioned include avocado oil, Camellia oil, turtle bean oil, macadamia nut oil, corn oil, mink oil, olive oil, Canoga oil, egg yolk oil, sesame seed oil, Persic oil, wheatgerm oil, Camellia sasanqua oil, castor oil, linseed oil, safflower oil, sunflower oil, grapeseed oil, apricot oil, shea oil, sweet almond oil, cotton oil, evening primrose oil, palm oil, perilla oil, hazelnut oil, soybean oil, peanut oil, tea seed oil, kaya oil, rice bran oil, rapeseed oil, alfalfa oil, Chinese tung tree wood oil, Japanese tung tree wood oil, jojoba oil, germ oil, poppy seed oil, pumpkin oil, blackcurrant oil, millet oil, barley oil, quinoa oil, rye oil, candlenut oil, passionflower oil, musk rose oil, triglycerine, glyceryl trioctanoate, and glyceryl triisopalmitate.

Solid oils/fats that can be mentioned include cocoa butter, coconut butter, horse fat, hardened coconut oil, palm oil, beef tallow, mutton tallow, hardened beef tallow, palm kernel oil, lard, Japan wax kernel oil, hardened oil, Japan wax, shea butter, and hardened castor oil.

Waxes that can be used include beeswax, candelilla wax, carnauba wax, lanolin, lanolin acetate, liquid lanolin, sugar cane wax, fatty acid isopropyl lanolin, hexyl laurate, reduced lanolin, jojoba wax, hard lanolin, polyoxyethylene (hereinafter referred to as POE), lanolin alcohol ether, POE lanolin alcohol acetate, lanolin fatty acid polyethylene glycol, and POE hydrogenated lanolin alcohol ether. In one embodiment the carrier is not lanolin based.

Ester oils that can be used include isopropyl myristate, cetyl octoate, octyldodecil myristate, isopropyl palmitate, butyl stearate, hexyl laurate, myristyl myristate, decyloleate, hexyldecyl dimethyl octoate, cetyl lactate, myristyl lactate, lanolin acetate, isocetyl stearate, isocetyl iso-stearate, 12-hydroxy cholesteryl stearate, di-2-ethylhexylic acid ethyleneglycol, dipentaerythritol fatty acid ester, N-alkylglycol monoisostearate, neopentylglycol dicaprate, diisostearyl malate, glyceryl di-2-heptyl undecanate, tri-methylol propane tri-2-ethylhexyl acid, tri-methylol propane triisostearate, pentaerythritol tetra-2-ethylhexyl acid, glyceryl tri-2-ethylhexanoate, tri-methylol propane triisostearate, cetyl-2-ethylexanoate, 2-ethylhexyl-palmitate, glycerine trimyristate, glyceride tri-2-heptyl undecatoic acid, methyl ester of castor oil fatty acid, oleate oil, acetoglyceride, palmitate-2-heptyl undecyl, diisopropyl adipate, N-lauroyl-L-glutamic acid-2-octyldodecil ester, di-2-heptylundecyl adipate, di-2-ethylhexyl sebacate, myristate-2-hexyldecyl, palmitate-2-hexyldecyl, adipate-2-hexyldecyl, diisopropyl sebacate, and succinate-2-ethylhexyl.

### xii. Higher Fatty Acids

Higher fatty acids that can be used include those mentioned elsewhere herein, including without limitation lauric acid, myristic acid, palmitic acid, stearic acid, behenic acid, oleic acid, 12-hydroxy-stearic acid, undecylenic acid, lanolin fatty acid, isostearic acid, linolic acid, linolenic acid, and eicosapentaenoic acid.

### xiii. Higher Alcohols

Higher alcohols of straight/branched chain that can be used include lauryl alcohol, cetyl alcohol, stearyl alcohol, behenyl alcohol, myristyl alcohol, oleyl alcohol, cetostearyl alcohol, monostearyl glycerine ether (batyl alcohol), 2-decyltetradecinol, lanolin alcohol, cholesterol, phytosterol, hexyldodecanol, isostearyl alcohol, octyldodecanol.

### xiv. Cosmetic Ingredients

Examples of cosmetic ingredient classes include: fragrances (artificial and natural; e.g. gluconic acid, phenoxyethanol, and triethanolamine), dyes and colorants (e.g., Blue 1, Blue 1 Lake, Red 40, Red 28 Lake, Red 7 Lake, Red 6 Lake, titanium dioxide, Unipure Red 6, Unipure Red 28, Unipure Red 33, Unipure Yellow OX, Unipure Yellow 5, FD&C blue 1, D&C blue no. 4, D&C green no. 5, D&C orange no. 4, D&C red no. 17, D&C red no. 6, D&C red no. 7, D&C red no. 30, D&C red no. 33, D&C violet no. 2, D&C yellow no. 10, D&C yellow no. 11, iron oxides, chromium oxides, tin oxide, ultramarines, and mica), flavoring agents (e.g. Stevia rebaudiana (sweetleaf) extract), adsorbents, lubricants, solvents (e.g. water, dimethyl isosorbide, hydrocarbons, C13-14 isoparaffin, hexylene glycol, isododecane, octyldodecanol, glycerin, denatured alcohol, 1,2-hexanediol, and propylene glycol), moisturizers (including, e.g., emollients, humectants, film formers, occlusive agents, and agents that affect the natural moisturization mechanisms of the skin), water-repellants, UV absorbers (physical and chemical absorbers such as paraaminobenzoic acid ("PABA") and corresponding PABA derivatives, titanium dioxide, zinc oxide, etc.), essential oils, vitamins (e.g. A, B, C, D, E, and K), trace metals (e.g. zinc, calcium and selenium), inorganic salts (e.g. sodium chloride, magnesium nitrate, and magnesium chloride), anti-irritants (e.g. steroids and non-steroidal anti-inflammatories), botanical extracts (e.g. aloe vera, chamomile, cucumber extract, ginkgo biloba, ginseng, and rosemary), anti-microbial agents, antioxidants (e.g., BHT and tocopherol), chelating agents (e.g., disodium EDTA and tetrasodium EDTA), preservatives (e.g., methylparaben and propylparaben), pH adjusters (e.g., sodium hydroxide, sodium citrate, triethanolamine, and citric acid), absorbents (e.g., aluminum starch octenylsuccinate, kaolin, corn starch, oat starch, cyclodextrin, talc, and zeolite), skin bleaching and lightening agents (e.g., hydroquinone and niacinamide lactate), humectants (e.g., sorbitol, urea, and manitol), exfoliants, waterproofing agents (e.g., magnesium/aluminum hydroxide stearate), conditioning agents (e.g., aloe extracts, allantoin, bisabolol, ceramides, dimethicone, hyaluronic acid, and dipotassium glycyrrhizate), and film formers (e.g. acrylates copolymer and polyquarternium-7).

### xv. UV Absorption Agents

UV absorption agents that can be used in the compositions include chemical and physical sunblocks. Non-limiting examples of chemical sunblocks that can be used include para-aminobenzoic acid (PABA), PABA esters (glyceryl PABA, amyldimethyl PABA and octyldimethyl PABA), butyl PABA, ethyl PABA, ethyl dihydroxypropyl PABA, benzophenones (oxybenzone, sulisobenzone, benzophenone, and benzophenone-1 through 12), cinnamates (octyl methoxycinnamate, isoamyl p-methoxycinnamate, octylmethoxy cinnamate, cinoxate, diisopropyl methyl cinnamate, DEA-methoxycinnamate, ethyl diisopropylcinnamate, glyceryl octanoate dimethoxycinnamate and ethyl methoxycinnamate), cinnamate esters, salicylates (homomethyl salicylate, benzyl salicylate, glycol salicylate, isopropylbenzyl salicylate, etc.), anthranilates, ethyl urocanate, homosalate, octisalate, oxtinoxate, dibenzoylmethane derivatives (e.g., avobenzone), octocrylene, octyl triazone, digalloy trioleate, glyceryl aminobenzoate, lawsone with dihydroxyacetone, ethylhexyl triazone, dioctyl butamido triazone, benzylidene malonate polysiloxane, terephthalylidene dicamphor sulfonic acid, disodium phenyl dibenzimidazole tetrasulfonate, diethylamino hydroxybenzoyl hexyl benzoate, bis diethylamino hydroxybenzoyl benzoate, bis benzoxazoylphenyl ethylhexylimino triazine, drometrizole trisiloxane, methylene bis-benzotriazolyl tetramethylbutyiphenol, and bis-ethylhexyloxyphenol methoxyphenyltriazine, 4-methylbenzylidenecamphor, and isopentyl 4-methoxycinnamate. Non-limiting examples of physical sunblocks include, kaolin, talc, petrolatum and metal oxides (e.g., titanium dioxide and zinc oxide).

### xvi. Moisturizing Agents

Non-limiting examples of moisturizing agents that can be used with the compositions include amino acids, chondroitin sulfate, diglycerin, erythritol, fructose, glucose, glycerin, glycerol polymers, glycol, 1,2,6-hexanetriol, honey, hyaluronic acid, hydrogenated honey, hydrogenated starch hydrolysate, inositol, lactitol, maltitol, maltose, mannitol, natural moisturizing factor, PEG-15 butanediol, polyglyceryl sorbitol, salts of pyrollidone carboxylic acid, potassium PCA, propylene glycol, sodium glucuronate, sodium PCA, sorbitol, sucrose, trehalose, urea, and xylitol.

Other examples include acetylated lanolin, acetylated lanolin alcohol, alanine, algae extract, aloe barbadensis, aloe-barbadensis extract, aloe barbadensis gel, althea officinalis extract, apricot (prunus armeniaca) kernel oil, arginine, arginine aspartate, arnica montana extract, aspartic acid, avocado (persea gratissima) oil, barrier sphingolipids, butyl alcohol, beeswax, behenyl alcohol, beta-sitosterol, birch (betula alba) bark extract, borage (borago officinalis) extract, butcherbroom (ruscus aculeatus) extract, butylene glycol, calendula officinalis extract, calendula officinalis oil, candelilla (euphorbia cerifera) wax, canola oil, caprylic/capric triglyceride, cardamon (elettaria cardamomum) oil, carnauba (copernicia cerifera) wax, carrot (daucus carota sativa) oil, castor (ricinus communis) oil, ceramides, ceresin, ceteareth-5, ceteareth-12, ceteareth-20, cetearyl octanoate, ceteth-20, ceteth-24, cetyl acetate, cetyl octanoate, cetyl palmitate, chamomile (anthemis nobilis) oil, cholesterol, cholesterol esters, cholesteryl hydroxystearate, citric acid, clary (salvia sclarea) oil, cocoa (theobroma cacao) butter, coco-caprylate/caprate, coconut (cocos nucifera) oil, collagen, collagen amino acids, corn (zea mays)oil, fatty acids, decyl oleate, dimethicone copolyol, dimethiconol, dioctyl adipate, dioctyl succinate, dipentaerythrityl hexacaprylate/hexacaprate, DNA, erythritol, ethoxydiglycol, ethyl linoleate, eucalyptus globulus oil, evening primrose (oenothera biennis) oil, fatty acids, geranium maculatum oil, glucosamine, glucose glutamate, glutamic acid, glycereth-26, glycerin, glycerol, glyceryl distearate, glyceryl hydroxystearate, glyceryl laurate, glyceryl linoleate, glyceryl myristate, glyceryl oleate, glyceryl stearate, glyceryl stearate SE, glycine, glycol stearate, glycol stearate SE, glycosaminoglycans, grape (vitis vinifera) seed oil, hazel (corylus americana) nut oil, hazel (corylus avellana) nut oil, hexylene glycol, hyaluronic acid, hybrid safflower (carthamus tinctorius) oil, hydrogenated castor oil, hydrogenated coco-glycerides, hydrogenated coconut oil, hydrogenated lanolin, hydrogenated lecithin, hydrogenated palm glyceride, hydrogenated palm kernel oil, hydrogenated soybean oil, hydrogenated tallow glyceride, hydrogenated vegetable oil, hydrolyzed collagen, hydrolyzed elastin, hydrolyzed glycosaminoglycans, hydrolyzed keratin, hydrolyzed soy protein, hydroxylated lanolin, hydroxyproline, isocetyl stearate, isocetyl stearoyl stearate, isodecyl oleate, isopropyl isostearate, isopropyl lanolate, isopropyl myristate, isopropyl palmitate, isopropyl stearate, isostearamide DEA, isostearic acid, isostearyl lactate, isostearyl neopentanoate, jasmine (jasminum officinale) oil, jojoba (buxus chinensis) oil, kelp, kukui (aleurites moluccana) nut oil, lactamide MEA, laneth-16, laneth-10 acetate, lanolin, lanolin acid, lanolin alcohol, lanolin oil, lanolin wax, lavender (lavandula angustifolia) oil, lecithin, lemon (citrus medica limonum) oil, linoleic acid, linolenic acid, macadamia ternifolia nut oil, maltitol, matricaria (chamomilla recutita) oil, methyl glucose sesquistearate, methylsilanol PCA, mineral oil, mink oil, mortierella oil, myristyl lactate, myristyl myristate, myristyl propionate, neopentyl glycol dicaprylate/dicaprate, octyldodecanol, octyldodecyl myristate, octyldodecyl stearoyl stearate, octyl hydroxystearate, octyl palmitate, octyl salicylate, octyl stearate, oleic acid, olive (olea europaea) oil, orange (citrus aurantium dulcis) oil, palm (elaeis guineensis) oil, palmitic acid, pantethine, panthenol, panthenyl ethyl ether, paraffin, PCA, peach (prunus persica) kernel oil, peanut (arachis hypogaea) oil, PEG-8 C12-18 ester, PEG-15 cocamine, PEG-150 distearate, PEG-60 glyceryl isostearate, PEG-5 glyceryl stearate, PEG-30 glyceryl stearate, PEG-7 hydrogenated castor oil, PEG-40 hydrogenated castor oil, PEG-60 hydrogenated castor oil, PEG-20 methyl glucose sesquistearate, PEG40 sorbitan peroleate, PEG-5 soy sterol, PEG-10 soy sterol, PEG-2 stearate, PEG-8 stearate, PEG-20 stearate, PEG-32 stearate, PEG40 stearate, PEG-50 stearate, PEG-100 stearate, PEG-150 stearate, pentadecalactone, peppermint (mentha piperita) oil, petrolatum, phospholipids, polyamino sugar condensate, polyglyceryl-3 diisostearate, polyquaternium-24, polysorbate 20, polysorbate 40, polysorbate 60, polysorbate 80, polysorbate 85, potassium myristate, potassium palmitate, propylene glycol, propylene glycol dicaprylate/dicaprate, propylene glycol dioctanoate, propylene glycol dipelargonate, propylene glycol laurate, propylene glycol stearate, propylene glycol stearate SE, PVP, pyridoxine dipalmitate, retinol, retinyl palmitate, rice (oryza sativa) bran oil, RNA, rosemary (rosmarinus officinalis) oil, rose oil, safflower (carthamus tinctorius) oil, sage (salvia officinalis) oil, sandalwood (santalum album) oil, serine, serum protein, sesame (sesamum indicum) oil, shea butter (butyrospermum parkii), silk powder, sodium chondroitin sulfate, sodium hyaluronate, sodium lactate, sodium palmitate, sodium PCA, sodium polyglutamate, soluble collagen, sorbitan laurate, sorbitan oleate, sorbitan palmitate, sorbitan sesquioleate, sorbitan stearate, sorbitol, soybean (glycine soja) oil, sphingolipids, squalane, squalene, stearamide MEA-stearate, stearic acid, stearoxy dimethicone, stearoxytrimethylsilane, stearyl alcohol, stearyl glycyrrhetinate, stearyl heptanoate, stearyl stearate, sunflower (helianthus annuus) seed oil, sweet almond (prunus amygdalus dulcis) oil, synthetic beeswax, tocopherol, tocopheryl acetate, tocopheryl linoleate, tribehenin, tridecyl neopentanoate, tridecyl stearate, triethanolamine, tristearin, urea, vegetable oil, water, waxes, wheat (triticum vulgare) germ oil, and ylang ylang (cananga odorata) oil.

### xvii. Antioxidants

Non-limiting examples of antioxidants that can be used with the compositions include acetyl cysteine, ascorbic acid polypeptide, ascorbyl dipalmitate, ascorbyl methylsilanol pectinate, ascorbyl palmitate, ascorbyl stearate, BHA, BHT, t-butyl hydroquinone, cysteine, cysteine HCl, diamylhydroquinone, di-t-butylhydroquinone, dicetyl thiodipropionate, dioleyl tocopheryl methylsilanol, disodium ascorbyl sulfate, distearyl thiodipropionate, ditridecyl thiodipropionate, dodecyl gallate, erythorbic acid, esters of ascorbic acid, ethyl ferulate, ferulic acid, gallic acid esters, hydroquinone, isooctyl thioglycolate, kojic acid, magnesium ascorbate, magnesium ascorbyl phosphate, methylsilanol ascorbate, natural botanical anti-oxidants such as green tea or grape seed extracts, nordihydroguaiaretic acid, octyl gallate, phenylthioglycolic acid, potassium ascorbyl tocopheryl phosphate, potassium sulfite, propyl gallate, quinones, rosmarinic acid, sodium ascorbate, sodium bisulfite, sodium erythorbate, sodium metabisulfite, sodium sulfite, superoxide dismutase, sodium thioglycolate, sorbityl furfural, thiodiglycol, thiodiglycolamide, thiodiglycolic acid, thioglycolic acid, thiolactic acid, thiosalicylic acid, tocophereth-5, tocophereth-10, tocophereth-12, tocophereth-18, tocophereth-50, tocopherol, tocophersolan, tocopheryl acetate, tocopheryl linoleate, tocopheryl nicotinate, tocopheryl succinate, and tris(nonylphenyl)phosphite.

### xviii. Structuring Agents

In other non-limiting aspects, the compositions can include a structuring agent. Structuring agent, in certain aspects, assist in providing rheological characteristics to the composition to contribute to the composition's stability. In other aspects, structuring agents can also function as an emollient, emulsifier or surfactant. Non-limiting examples of structuring agents include stearic acid, palmitic acid, stearyl alcohol, cetyl alcohol, PPG-30 cetyl ether, behenyl alcohol, stearic acid, palmitic acid, the polyethylene glycol ether of stearyl alcohol having an average of about 1 to about 21 ethylene oxide units, the polyethylene glycol ether of cetyl alcohol having an average of about 1 to about 5 ethylene oxide units, polyoxyethylene methylglucoside dioleate, tea-lauryl sulfate, polyethylene glycol ester of stearic acid, C.sub.12-15 alkyl benzoate, propylene glycol myristyl ether acetate, 3-hydroxypropyl (E)-octadec-9-enoate, sorbitan laurate, sorbitan stearate, carbomer, ammonium acryloyldimethyltaurate/carboxyethyl acrylate crosspolymer, sodium laureth sulfate, hydroxypropyl cyclodextrin, PPG-26 oleate, dimethicone/PEG-10/15 crosspolymer, and mixtures thereof.

### xix. Emulsifiers

In certain aspects of the present invention, the compositions do not include an emulsifier. In other aspects, however, the compositions can include one or more emulsifiers. Emulsifiers can reduce the interfacial tension between phases and improve the formulation and stability of an emulsion. The emulsifiers can be nonionic, cationic, anionic, and zwitterionic emulsifiers (See McCutcheon's (1986); U.S. Pat. Nos. 5,011,681; 4,421,769; 3,755,560). Non-limiting examples include esters of glycerin, esters of propylene glycol, fatty acid esters of polyethylene glycol, fatty acid esters of polypropylene glycol, esters of sorbitol, esters of sorbitan anhydrides, hydrolyzed Attorney Docket No. 07341.006WO1 jojoba esters, carboxylic acid copolymers, esters and ethers of glucose, ethoxylated ethers, ethoxylated alcohols, alkyl phosphates, polyoxyethylene fatty ether phosphates, fatty acid amides, acyl lactylates, soaps, TEA stearate, DEA oleth-3 phosphate, polyethylene glycol 20 sorbitan monolaurate (polysorbate 20), polyethylene glycol 5 soya sterol, steareth-2, steareth-20, steareth-21, ceteareth-20, PPG-2 methyl glucose ether distearate, ceteth-10, polysorbate 80, cetyl phosphate, potassium cetyl phosphate, diethanolamine cetyl phosphate, polysorbate 60, glyceryl stearate, PEG-100 stearate, C20-40 alcohols, PEG/PPG-18/18 dimethicone, maltodextrin, sodium polyacrylate, and mixtures thereof.

### xx. Silicone Containing Compounds

In non-limiting aspects, silicone containing compounds include any member of a family of polymeric products whose molecular backbone is made up of alternating silicon and oxygen atoms with side groups attached to the silicon atoms. By varying the --Si--O-- chain lengths, side groups, and crosslinking, silicones can be synthesized into a wide variety of materials. They can vary in consistency from liquid to gel to solids.

The silicone containing compounds that can be used include those described in this specification or those known to a person of ordinary skill in the art. Non-limiting examples include silicone oils (e.g., volatile and non-volatile oils), gels, and solids. In certain aspects, the silicon containing compounds includes a silicone oils such as a polyorganosiloxane. Non-limiting examples of polyorganosiloxanes include dimethicone, cyclomethicone, polysilicone-11, phenyl trimethicone, trimethylsilylamodimethicone, stearoxytrimethylsilane, or mixtures of these and other organosiloxane materials in any given ratio in order to achieve the desired consistency and application characteristics depending upon the intended application (e.g., to a particular area such as the skin, hair, or eyes). A "volatile silicone oil" includes a silicone oil have a low heat of vaporization, i.e. normally less than about 50 cal per gram of silicone oil. Non-limiting examples of volatile silicone oils include: cyclomethicones such as Dow Corning 344 Fluid, Dow Corning 345 Fluid, Dow Corning 244 Fluid, and Dow Corning 245 Fluid, Volatile Silicon 7207 (Union Carbide Corp., Danbury, Conn.); low viscosity dimethicones, i.e. dimethicones having a viscosity of about 50 mm²/s (50 cSt) or less (e.g., dimethicones such as Dow Corning 200-0.5 cst Fluid). The Dow Corning Fluids are available from Dow Corning Corporation, Midland, Mich. Cyclomethicone and dimethicone are described in the Third Edition of the CTFA Cosmetic Ingredient Dictionary as cyclic dimethyl polysiloxane compounds and a mixture of fully methylated linear siloxane polymers end-blocked with trimethylsiloxy units, respectively. Silicone containing compounds of the invention may also be used as bulking agents (e.g. silicic acid and aluminum calcium sodium silicate). Other non-limiting volatile silicone oils that can be used in the context of the present invention include those available from General Electric Co., Silicone Products Div., Waterford, N.Y. and SWS Silicones Div. of Stauffer Chemical Co., Adrian, Mich.

### xxi. Preservatives

Non-limiting examples of preservatives that can be used include quaternary ammonium preservatives such as polyquaternium-1 and benzalkonium halides (e.g., benzalkonium chloride ("BAC") and benzalkonium bromide), parabens (e.g., methylparabens and propylparabens), benzyl alcohol, chlorobutanol, phenol, sorbic acid, thimerosal, caprylyl glycol, iodopropynyl butylcarbamate, methylisothiazolinone, methylchloroisothiazolinone, sodium benzoate, dimethylol-5,5-dimethylhydantoin, 3-iodo-2-propynyl butyl carbamate, phenoxyethanol, caprylyl alcohol, ethylhexyl glycerin, hexylene glycol, DMDM hydantoin, chlorphenesin, and combinations thereof.

### xxii. Conditioning Agents

Non-limiting examples of conditioning agents that can be used include caprylyl glycol, ethylhexylglycerin, PEG-12 dimethicone, hydroxypropyl cyclodextrin, dimethicone, tocopheryl acetate, Butyrospermum parkii (shea butter), polymers of polyethylene glycol and methicone, Helianthus annuus (sunflower) seed oil, Euterpe oleracea fruit extract, Camellia oleifera leaf extract, hydrolyzed Myrtus communis leaf extract, PEG-18 glyceryl oleate/cocoate, cyclotetrasiloxane, cyclohexasiloxane, cyclopentasiloxane, tocopherol, glycerin, Undaria pinnatifida extract, Carthamus tinctorius (safflower) oleosomes, butylene glycol, Myrciaria dubia fruit extract, Castanea sativa (chestnut) seed extract, allantoin, hydrogenated palm kernel oil, caprylic/capric triglyceride, propylene glycol stearate, panthenol, polypropylene glycol ether of cetyl alcohol, polyquaternium-7, ethoxylated glyceryl esters, ethylhexyl palmitate. aloe extracts, bisabolol, ceramides, hyaluronic acid, dipotassium glycyrrhizate, cocamidopropyl betaine, pentaerythrityl tetraisostearate, glyceryl behenate/eicosadioate, tridecyl trimellitate, salicylic acid, dimethicone/vinyl dimethicone crosspolymer; PEG-9 dimethicone, biosaccharide gum, decylene glycol, ethylene brassylate, pentylene glycol, polyglyceryl-10 laurate, ionone, tetramethyl acetyloctahydronaphthalenes, methyl decenol, dimethyl heptenal, trimethylcyclopentenyl dimethylisopentenol, 3-hexenol, jojoba esters, PEG-8 dimethicone, and mixtures thereof.

### xxiii. Additional Components

Additional components of the compositions are understood by those of skill in the art and may generally be found in Remington, The Science and Practice of Phannacy, Gennaro A. ed., p. 681-699, 20th Edition, Lippincott, 2000.

### II. PROCESSES FOR MAKING

Compositions of the present invention can be prepared by any technique known to a person of ordinary skill in the art of pharmacy, pharmaceutics, drug delivery, pharmacokinetics, medicine or other related discipline that comprises admixing one or more excipients with a therapeutic agent to form a composition, drug delivery system or component thereof.

In another aspect, a process is provided for making a transdermal and/or dermal composition comprising a lipophilic active agent in an oil-in-water emulsion comprising an aqueous phase and an oil phase, the steps comprising:
i) combining a therapeutically effective amount of the lipophilic active agent with an edible oil comprising long chain fatty acids and/or medium chain fatty acids;
ii) dehydrating the product of step (i), thereby producing a dehydrated mixture; and
iii) combining the aqueous phase with the oil phase, wherein the oil phase comprises the dehydrated mixture of step (ii).
In other aspects, the bioavailability of the lipophilic active agent in a subject is at least 2 times greater than the bioavailability of the lipophilic active agent in the subject in the absence of the edible oil comprising long chain fatty acids and/or medium chain fatty acids, particularly at least 5 times greater, and more particularly at least 10 times greater. In another aspect, the edible oil comprising long chain fatty acids and/or medium chain fatty acids is substantially free of omega-6 fatty acids. In another aspect, the lipophilic active agent is selected from the group consisting of: cannabinoids, terpenes and terpenoids, non-steroidal anti-inflammatory drugs (NSAIDs), vitamins, nicotine, phosphodiesterase 5 (PDE5) inhibitors, Maca extract, hormones, fentanyl, buprenorphine, scopolamine, and antioxidants.

### III. METHODS OF TREATMENT

Although not according to the invention, a method of treating one or more conditions is described comprising administering any of the compositions disclosed herein to a subject in need thereof. For example, a method of treating a condition by administering a composition disclosed herein topically.

For illustrative purposes, for example the condition may be, without limitation, inflammation, colorectal polyps, menstrual cramps, sports injuries, osteoarthritis, rheumatoid arthritis, and pain, *e.g*., acute pain, or of reducing the risk of peptic ulceration.

As used herein, the term "subject" treated by the presently disclosed methods in their many aspects is desirably a human subject, although it is to be understood that the methods described herein are effective with respect to all vertebrate species, which are intended to be included in the term "subject." Accordingly, a "subject" can include a human subject for medical purposes, such as for the diagnosis or treatment of an existing disease, disorder, condition or the prophylactic diagnosis or treatment for preventing the onset of a disease, disorder, or condition or an animal subject for medical, veterinary purposes, or developmental purposes. Suitable animal subjects include mammals including, but not limited to, primates, e.g., humans, monkeys, apes, gibbons, chimpanzees, orangutans, macaques and the like; bovines, e.g., cattle, oxen, and the like; ovines, e.g., sheep and the like; caprines, e.g., goats and the like; porcines, e.g., pigs, hogs, and the like; equines, e.g., horses, donkeys, zebras, and the like; felines, including wild and domestic cats; canines, including dogs; lagomorphs, including rabbits, hares, and the like; and rodents, including mice, rats, guinea pigs, and the like. An animal may be a transgenic animal. In some aspects, the subject is a human including, but not limited to, fetal, neonatal, infant, juvenile, and adult subjects. Further, a "subject" can include a patient afflicted with or suspected of being afflicted with a disease, disorder, or condition. Thus, the terms "subject" and "patient" are used interchangeably herein. Subjects also include animal disease models (e.g., rats or mice used in experiments, and the like).

The term "effective amount," as in "a therapeutically effective amount," of a therapeutic agent refers to the amount of the agent necessary to elicit the desired biological response. As will be appreciated by those of ordinary skill in this art, the effective amount of an agent may vary depending on such factors as the desired biological endpoint, the agent to be delivered, the composition of the pharmaceutical composition, the target tissue or cell, and the like. More particularly, the term "effective amount" refers to an amount sufficient to produce the desired effect, e.g., to reduce or ameliorate the severity, duration, progression, or onset of a disease, disorder, or condition, or one or more symptoms thereof; prevent the advancement of a disease, disorder, or condition, cause the regression of a disease, disorder, or condition; prevent the recurrence, development, onset or progression of a symptom associated with a disease, disorder, or condition, or enhance or improve the prophylactic or therapeutic effect(s) of another therapy.

Actual dosage levels of the active ingredients in the presently disclosed compositions can be varied so as to obtain an amount of the active ingredient that is effective to achieve the desired therapeutic response for a particular subject, composition, route of administration, and disease, disorder, or condition without being toxic to the subject. The selected dosage level will depend on a variety of factors including the activity of the particular composition employed, the route of administration, the time of administration, the rate of excretion of the particular composition being employed, the duration of the treatment, other drugs, and/or materials used in combination with the particular composition employed, the age, sex, weight, condition, general health and prior medical history of the patient being treated, and like factors well known in the medical arts.

A physician having ordinary skill in the art can readily determine and prescribe the effective amount of the presently disclosed composition required. Accordingly, the dosage range for administration may be adjusted by the physician as necessary, as described more fully elsewhere herein.

### A. Skin Conditions

Although not according to the invention, a method of treatment or prevention of a skin condition is described, comprising the topical application of the composition according to the invention herein onto the skin of a subject afflicted with the skin condition, or at risk of being afflicted with the skin condition. The skin condition may be selected from the group comprising skin ageing, elastosis, laxity (sagging), rhytids (wrinkles), skin infection, skin damage, skin burn, pain, and muscle tightness, and combinations thereof.

For instance, the compositions can be used to treat or prevent a fine line or wrinkle, erythema, sensitive skin, or inflamed skin. In particular aspects, erythema, sensitive skin, or inflamed skin is caused by skin sunburn, electrical treatments of skin, skin burns, contact allergies, systemic allergies, skin toxicity, exercise, insect stings, bacterial infection, viral infection, fungal infection, protozoa infection, massage, or windburn. In other aspects, the following additional skin conditions can be treated or prevented in accordance with the methods and compositions disclosed throughout the specification and claims: pruritus, lentigo, spider veins, age spots, senile purpura, keratosis, melasma, blotches, nodules, sun damaged skin, dermatitis (including, but not limited to seborrheic dermatitis, nummular dermatitis, contact dermatitis, atopic dermatitis, exfoliative dermatitis, perioral dermatitis, and stasis dermatitis), psoriasis, folliculitis, rosacea, acne, impetigo, erysipelas, erythrasma, eczema, and other inflammatory skin conditions.

In certain non-limiting aspects, the skin condition can be caused by exposure to UV light, age, irradiation, chronic sun exposure, environmental pollutants, air pollution, wind, cold, heat, chemicals, disease pathologies, smoking, or lack of nutrition. The skin can be facial skin or non-facial skin (e.g., arms, legs, hands, chest, back, feet, etc.).

The method can further comprise identifying a person in need of skin treatment. The person can be a male or female. The age of the person can be at least 1, 2, 3, 4, 5, 6, 7, 8, 9, 10, 15, 20, 25, 30, 35, 40, 45, 50, 55, 60, 65, 70, 75, 80, 85, 90, 95, or more years old, or any range derivable therein.

The method can also include topically applying an amount effective to: increase the stratum corneum turnover rate of the skin; increase collagen synthesis in fibroblasts; increase cellular anti-oxidant defense mechanisms (e.g., exogenous additions of anti-oxidants can bolster, replenish, or prevent the loss of cellular antioxidants such as catalase and glutathione in skin cells (e.g., keratinocytes, melanocytes, langerhans cells, etc.) which will reduce or prevent oxidative damage to the skin, cellular, proteins, and lipids); inhibit melanin production in melanocytes; reduce or prevent oxidative damage to skin (including reducing the amount lipid peroxides and/or protein oxidation in the skin).

Although not according to the invention, a cosmetic treatment of the skin is described, comprising the application of the composition according to the invention herein on the skin. The cosmetic treatment may be for alleviating or preventing any of the disorders described above and combinations thereof.

### B. Cannabinoids

In one aspect, since the lipophilic active agent within the compositions of the invention is a cannabinoid, the one or more conditions is selected from the group consisting of cardiac diseases such as heart disease, ischemic infarcts, and cardiometabolic disorders; neurological diseases such as Alzheimer's disease, Parkinson's disease, schizophrenia, and Human Immunodeficiency Virus (HIV) dementia; obesity; metabolic disorders such as insulin related deficiencies and lipid profiles, hepatic diseases, diabetes, and appetite disorders; cancer chemotherapy; benign prostatic hypertrophy; irritable bowel syndrome; biliary diseases; ovarian disorders; marijuana abuse; and alcohol, opioid, nicotine, or cocaine addiction; and sexual dysfunction such as erectile dysfunction.

### IV. KITS AND CONTAINERS

Also contemplated are kits that include any one of the compositions disclosed throughout the specification and claims. In certain embodiments, the composition is comprised in a container. The container can be a bottle, dispenser, or package. The container can dispense a predetermined amount of the composition. In certain aspects, the compositions is dispensed in a spray, dollop, or liquid. The container can include indicia on its surface. The indicia can be a word, an abbreviation, a picture, or a symbol.

Also contemplated is a product comprising a composition of the present invention. In non-limiting aspects, the product can be a cosmetic product. The cosmetic product can be those described in other sections of this specification or those known to a person of skill in the art. Non-limiting examples of products include a moisturizer, a cream, a lotion, a skin softener, a foundation, a night cream, a lipstick, a cleanser, a toner, a sunscreen, a mask, or an anti-aging product.

Also contemplated are containers comprising a composition of the present invention. In non-limiting aspects, the container can be a bottle, a metal tube, a laminate tube, a plastic tube, a dispenser, a pressurized container, a barrier container, a package, a compartment, a lipstick container, a compact container, cosmetic pans that can hold cosmetic compositions, or other types of containers such as injection or blow-molded plastic containers into which the dispersions or compositions or desired bottles, dispensers, or packages are retained. The containers can have spray, pump, or squeeze mechanisms.

Although not according to the invention, there is described a face mask incorporating the composition according to the invention herein. The face mask may comprise a cream, lotion or paste. The face mask may be provided in the form of a composition that is to be applied directly to the skin to form a mask. The face mask may alternatively be provided in the form of a wrap impregnated or soaked in the composition, wherein the wrap is to be applied onto the skin.

### EXAMPLES

### Example 1

The purpose of this experiment was to prepare "oil-in-water" emulsion creams as test articles for skin permeability evaluation as described in Example 2 below.

As used herein, transdermal and/or dermal compositions incorporating DEHYDRATECH^{™} are transdermal and/or dermal compositions that incorporate a dehydrated mixture comprising a therapeutically effective amount of a lipophilic active agent and an edible oil comprising long chain fatty acids and/or medium chain fatty acids, particularly wherein dehydrated mixture is obtainable by the steps of:
i) combining a therapeutically effective amount of the lipophilic active agent
   with the edible oil comprising long chain fatty acids and/or medium chain fatty acids; and
ii) dehydrating the product of step (i), thereby producing the dehydrated mixture.

Six test articles were prepared as follows for comparative permeability testing:

### TEST ARTICLE A (WITH DEHYDRATECH^{™})

Dehydration Step: Cetosteryl Alcohol (8g) and Glyceryl Monostearate (2g) were combined in a bowl, and mixed with a combination of 90% Hemp Oil (333 mg = 300 mg CBD) and Sunflower Oil (600 mg) (heated to lower than 50°C before addition) to create a Sunflower Oil mixture. The Sunflower Oil mixture was placed in a dehydrator at 51.67°C (125°F) for 90 minutes. The Sunflower Oil mixture was removed from the dehydrator after 45 minutes to stir and then returned to the dehydrator for the remaining 45 minutes, thereby creating a dehydrated Sunflower Oil mixture.

Aqueous Phase: Polysorbate 60 (3.6 g), Glycerine (30 g), and water (41.7g) were mixed and heated to 65°C to create an Aqueous Phase.

Oil Phase: In a separate vessel, Liquid paraffin (5 g) and White Soft Paraffin (10g) were mixed and heated to 71-75°C with the dehydrated Sunflower Oil mixture (*i*.*e*., the dehydrated mixture of Cetosteryl Alcohol, Glyceryl Monostearate, 90% Hemp Oil containing 300 mg CBD, and Sunflower Oil), thereby creating an Oil Phase.

The Oil Phase was added to the Aqueous Phase with stirring to maintain a temperature of 65°C for 15 minutes before cooling to room temperature, thereby creating Test Article A.

### TEST ARTICLE B (WITH DEHYDRATECH^{™} AND TRANSCUTOL^{®})

Dehydration Step: Cetosteryl Alcohol (8g) and Glyceryl Monostearate (2g) were combined in a bowl, and mixed with a combination of 90% Hemp Oil (333 mg = 300 mg CBD) and Sunflower Oil (600 mg) (heated to lower than 50°C before addition) to create a Sunflower Oil mixture. The Sunflower Oil mixture was placed in a dehydrator at 51.67°C (125°F) for 90 minutes. The Sunflower Oil mixture was removed from the dehydrator after 45 minutes to stir and then returned to the dehydrator for the remaining 45 minutes, thereby creating a dehydrated Sunflower Oil mixture.

Aqueous Phase: Polysorbate 60 (3.6 g), Glycerine (30 g), and water (41.7g) were mixed and heated to 65°C to create an Aqueous Phase.

Oil Phase: In a separate vessel, Liquid paraffin (5 g) and White Soft Paraffin (10g) were mixed and heated to 71-75°C, thereby creating an Oil Phase.

The Oil Phase was added to the Aqueous Phase with stirring to maintain a temperature of 65°C for 15 minutes before cooling to room temperature. Prior to cooling, the dehydrated Sunflower Oil mixture (*i*.*e*., the dehydrated mixture of Cetosteryl Alcohol, Glyceryl Monostearate, 90% Hemp Oil containing 300 mg CBD, and Sunflower Oil) was mixed into 10 grams of TRANSCUTOL^{®} (diethylene glycol monoethyl ether) at room temperature and then added into the cooling biphasic mixture of Aqueous and Oil Phases at 40°C. Thereby creating Test Article B.

### TEST ARTICLE C (WITH DEHYDRATECH^{™} AND CAPRYOL^{™})

Dehydration Step: Cetosteryl Alcohol (8g) and Glyceryl Monostearate (2g) were combined in a bowl, and mixed with a combination of 90% Hemp Oil (333 mg = 300 mg CBD) and Sunflower Oil (600 mg) (heated to lower than 50°C before addition) to create a Sunflower Oil mixture. The Sunflower Oil mixture was placed in a dehydrator at 51.67°C (125°F) for 90 minutes. The Sunflower Oil mixture was removed from the dehydrator after 45 minutes to stir and then returned to the dehydrator for the remaining 45 minutes, thereby creating a dehydrated Sunflower Oil mixture.

Aqueous Phase: Polysorbate 60 (3.6 g), Glycerine (30 g), and water (41.7g) were mixed and heated to 65°C to create an Aqueous Phase.

Oil Phase: In a separate vessel, Liquid paraffin (5 g) and White Soft Paraffin (10g) were mixed and heated to 71-75°C, thereby creating an Oil Phase.

The dehydrated Sunflower Oil mixture (*i*.*e*., the dehydrated mixture of Cetosteryl Alcohol, Glyceryl Monostearate, 90% Hemp Oil containing 300 mg CBD, and Sunflower Oil) was mixed into 10 grams of CAPRYOL^{™} (propylene glycol monocaprylate) at room temperature, and then added into the Oil Phase while it is at 71-75°C.

The Oil Phase (mixed with the dehydrated Sunflower Oil mixture) was added to the Aqueous Phase with stirring to maintain a temperature of 65°C for 15 minutes before cooling to room temperature, thereby creating Test Article C.

### TEST ARTICLE D (WITHOUT DEHYDRATECH^{™}/WITH TRANSCUTOL^{®})

Aqueous Phase: Polysorbate 60 (3.6 g), Glycerine (30 g), and water (41.7g) were mixed and heated to 65°C to create an Aqueous Phase.

Oil Phase: In a separate vessel, Liquid paraffin (5 g), White Soft Paraffin (10g), Cetosteryl Alcohol (8g), and Glyceryl Monostearate (2g) were mixed and heated to 71-75°C, thereby creating an Oil Phase.

The Oil Phase was added to the Aqueous Phase with stirring to maintain a temperature of 65°C for 15 minutes before cooling to room temperature. 90% Hemp Oil (333 mg = 300 mg CBD) was mixed into 10 grams of TRANSCUTOL^{®} (diethylene glycol monoethyl ether) at room temperature, and then added into the cooling biphasic mixture at 40°C. Thereby creating Test Article D.

### TEST ARTICLE E (WITHOUT DEHYDRATECH^{™}/WITH CAPRYOL^{™})

Aqueous Phase: Polysorbate 60 (3.6 g), Glycerine (30 g), and water (41.7g) were mixed and heated to 65°C to create an Aqueous Phase.

Oil Phase: In a separate vessel, Liquid paraffin (5 g), White Soft Paraffin (10g), Cetosteryl Alcohol (8g), and Glyceryl Monostearate (2g) were mixed and heated to 71-75°C, thereby creating an Oil Phase.

The Oil Phase was added to the Aqueous Phase with stirring to maintain a temperature of 65°C for 15 minutes before cooling to room temperature. 90% Hemp Oil (333 mg = 300 mg CBD) was mixed into 10 grams of CAPRYOL^{™} (propylene glycol monocaprylate) at room temperature, and then added into the cooling biphasic mixture at 40°C. Thereby creating Test Article E.

### TEST ARTICLE F (WITHOUT DEHYDRATECH^{™}, TRANSCUTOL^{®}, OR CAPRYOL^{™})

Aqueous Phase: Polysorbate 60 (3.6 g), Glycerine (30 g), and water (41.7g) were mixed and heated to 65°C to create an Aqueous Phase.

Oil Phase: In a separate vessel, Liquid paraffin (5 g), White Soft Paraffin (10g), Cetosteryl Alcohol (8g), Glyceryl Monostearate (2g), and 90% Hemp Oil (333 mg = 300 mg CBD) were mixed and heated to 71-75°C, thereby creating an Oil Phase.

The Oil Phase was added to the Aqueous Phase with stirring to maintain a temperature of 65°C for 15 minutes before cooling to room temperature, thereby creating Test Article F.

### Example 2

A liquid chromatography - tandem mass spectrometry ("LC-MS/MS") analytical method was implemented for Test Articles A through F from Example 1, and CBD skin delivery and permeation were measured using human cadaver skin in Franz diffusion cells ("FDC"s).

Skin from a single donor was shipped and stored frozen at -20°C until needed. The skin was removed from the freezer, allowed to equilibrate to room temperature, and then cut to ~2 cm x 2 cm pieces before testing. FDCs with a 3.3 ml receiver volume and 0.55 cm² diffusional area were employed.

De-aerated isotonic phosphate buffered saline solution at pH 7.4 ("PBS") containing 0.01% NaN3 (a preservative) and 4% hydroxypropyl-β-cyclodextrin ("HPBCD"), PEG400 or Brij98 was verified as a suitable solvent system for the receptor well medium for the CBD (the "Receptor Fluid"). Dimethyl sulfoxide ("DMSO") was verified as a suitable solvent for extracting CBD from epidermal and dermal tissues (the "Extraction Fluid").

Receptor wells were filled with Receptor Fluid. Pieces of skin were mounted on the receptor cells, the conventional donor wells applied, and the assembly clamped together with uniform pressure using a pinch clamp. After assembly of the FDC, the skin was allowed to hydrate for 20 minutes in contact with the receptor fluid. Any FDCs that evidenced any leakage during this period were discarded.

The integrity and quality of the tissue was tested prior to application of the test formulations through measurement of transepidermal electrical resistance ("TEER"). Skin pieces that evidence TEER values that differed substantially from the mean of all FDCs were discarded and the TEER value of accepted tissue pieces were used to guide the distribution of formulation samples over the tissue piece set.

Six (6) replicates of each formulation (Test Articles A through F from Example 1) were examined in a batch of FDC's. Each test solution was applied at a finite dose of 5µL and spread uniformly over the addressed skin surface area.

The receptor well was maintained at 32°C and the Receptor Fluid in the receptor wells were stirred with a magnetic stir bar throughout the experiments. A sample was abstracted from each receptor well at each of 4h, 10h, 24h and 48h, and the receptor well was replenished with fresh Receptor Fluid.

The concentration of CBD in each receptor well sample was assayed by a verified LC-MS/MS analytical method.

At the end of the experiment (48h) remnant formulation (if any) was removed by wiping the skin exterior twice with a 4x4cm piece of Kimwipe^{™} soaked with EtOH-Water 50-50 (the "Washing Fluid"), and the skin then wiped dry with a Kimwipe^{™}.

The successive topmost layers of the stratum corneum were removed by four (4) times applying cellophane tape to the skin and then pulling off the tape. Material in these tape strips will be considered only superficially absorbed and the tape strips were discarded.

The epidermal and dermal layers were separated, using mild heating if required.

The epidermal and dermal sections for each FDC were placed into 4ml glass vials. 2ml of the Extraction Fluid, was added to each vial and the vials allowed to incubate for 24h. At the end of the extraction period, aliquots of the Extraction Fluid were drawn and filtered.

The CBD concentrations in the epidermal and dermal tissue extracts were analyzed by the verified LC-MS/MS method. Results are shown in Figures 1 to 4.

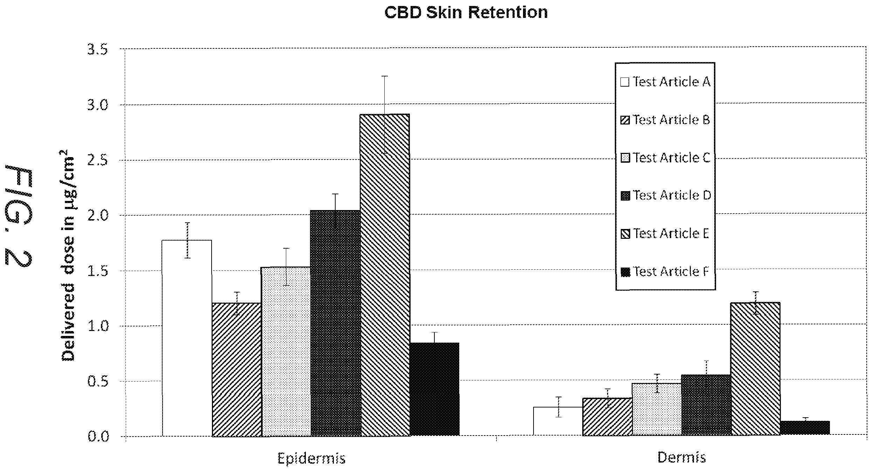

Figure 1 shows transdermal delivery of CBD in µg/cm² for Test Articles A through F, at 2h, 4h, 10h, 24h, and 48 hr, as well as skin retention in the epidermis and dermis (see Figure 2).

Figure 2 shows skin retention of CBD in µg/cm² for Test Articles A through F in the epidermis and dermis.

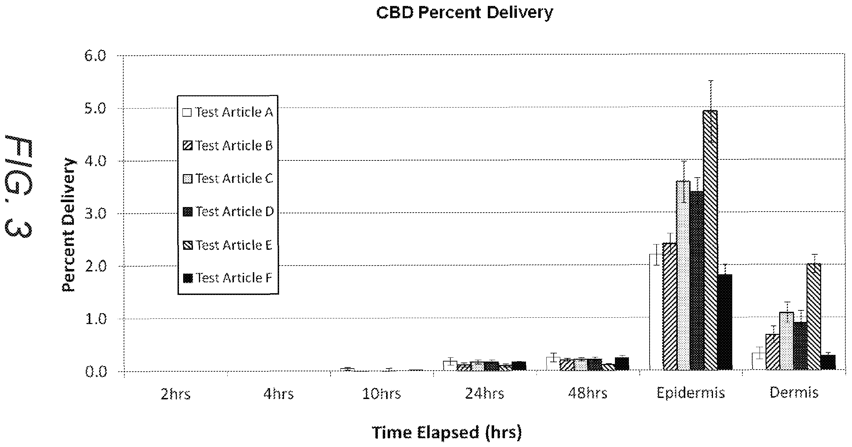

Figure 3 shows CBD percent delivery for Test Articles A through F, at 2h, 4h, 10h, 24h, and 48 hr, as well as in the epidermis and dermis.

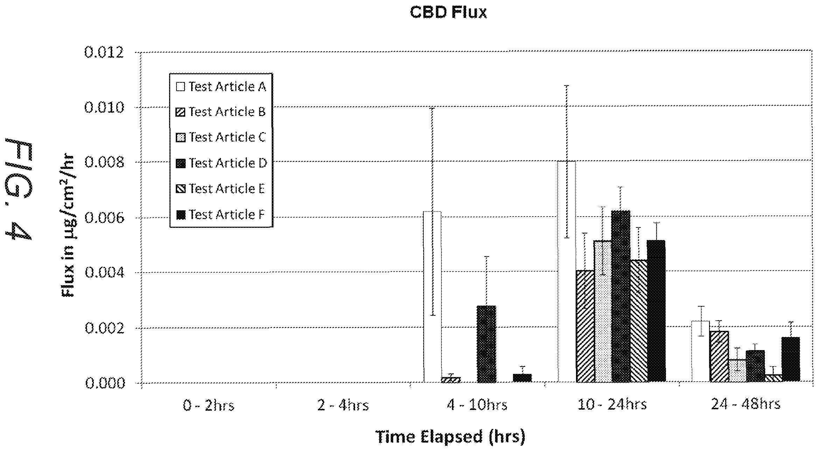

Figure 4 shows CBD Flux in µg/cm²/hr for Test Articles A through F, at 0h-2h, 2h-4h, 4h-10h, 10h-24h, and 24h-48h.

As shown in Figures 1 and 2, Test Article A outperformed Test Article F, demonstrating that the DEHYDRATECH^{™} formulation provided unexpectedly superior properties relative to the control formulation without DEHYDRATECH^{™}. Without being bound by theory, it is thought that this superior transdermal penetration may be due to emollient properties of long chain fatty acids from the sunflower oil integrated into the formulation of Test Article A.

Also as shown in Figures 1 and 2, of the penetration enhancers included in the formulations of Test Articles B and D (TRANSCUTOL^{®}, or diethylene glycol monoethyl ether, with and without the DEHYDRATECH^{™} formulation) and Test Articles C and E (CAPRYOL^{™}, or propylene glycol monocaprylate, with and without the DEHYDRATECH^{™} formulation), CAPRYOL^{™} (propylene glycol monocaprylate) yielded better penetration enhancement data with DEHYDRATECH^{™} than TRANSCUTOL^{®} (diethylene glycol monoethyl ether). This effect was observed both in terms of epidermis delivery (i.e., into the skin) and dermis delivery (i.e., deeper through the skin into the typically vascularized compartment). However, it was surprising that the DEHYDRATECH^{™} formulation without the addition of TRANSCUTOL^{®} (diethylene glycol monoethyl ether) or CAPRYOL^{™} (Test Article A) was a powerful delivery system all on its own compared to the control formulation that lacked added penetration enhancer components or the DEHYDRATECH^{™} formulation (Test Article F).

As shown in Figure 3, CBD percent delivery data showed a pronounced permeability enhancement for Test Article A (DEHYDRATECH^{™} formulation) compared to Test Article F (the control formulation that lacked added penetration enhancer components or the DEHYDRATECH^{™} formulation).

The CBD Flux data of Figure 4 represents the amount of CBD driven into the theoretical systemic circulation fraction. As shown in Figure 4, Test Article A (DEHYDRATECH^{™} formulation lacking any added penetration enhancers) was surprisingly superior to the other tested formulations. In other words, the use of a dehydrated substrate base (cetosteryl alcohol, glyceryl monostearate, sunflower oil, hemp oil) unexpectedly produced a significant increase in systemic CBD permeability, even without the inclusion of the penetration enhancers TRANSCUTOL^{®} (diethylene glycol monoethyl ether) or CAPRYOL^{™} (propylene glycol monocaprylate).

All publications, patent applications, patents, and other references mentioned in the specification are indicative of the level of those skilled in the art to which the presently disclosed subject matter pertains. It will be understood that, although a number of patent applications, patents, and other references are referred to herein, such reference does not constitute an admission that any of these documents forms part of the common general knowledge in the art.

Although the foregoing subject matter has been described in some detail by way of illustration and example for purposes of clarity of understanding, it will be understood by those skilled in the art that certain changes and modifications can be practiced within the scope of the appended claims.

## Claims

1. A transdermal and/or dermal composition comprising a lipophilic active agent in an oil-in-water emulsion comprising an aqueous phase and an oil phase, wherein the composition is obtainable by providing an oil phase comprising a dehydrated mixture, and wherein the dehydrated mixture comprises a therapeutically effective amount of the lipophilic active agent and an edible oil comprising long chain fatty acids and/or medium chain fatty acids, wherein the lipophilic active agent is cannabidiol and the edible oil is sunflower oil, and wherein the dehydrated mixture is obtainable by the steps of:
i) combining the therapeutically effective amount of the cannabidiol with the sunflower oil; and
ii) dehydrating the product of step (i), thereby producing the dehydrated mixture;
and combining the oil phase with an aqueous phase to provide the oil-in-water emulsion.

2. The transdermal and/or dermal composition claim 1, further comprising an emulsifier, wherein the emulsifier is selected from: esters of glycerin, esters of propylene glycol, fatty acid esters of polyethylene glycol, fatty acid esters of polypropylene glycol, esters of sorbitol, esters of sorbitan anhydrides, hydrolyzed jojoba esters, carboxylic acid copolymers, esters and ethers of glucose, ethoxylated ethers, ethoxylated alcohols, alkyl phosphates, polyoxyethylene fatty ether phosphates, fatty acid amides, acyl lactylates, soaps, triethylamine (TEA) stearate, diethylamine (DEA) oleth-3 phosphate, polyethylene glycol 20 sorbitan monolaurate (polysorbate 20), polyethylene glycol 5 soya sterol, steareth-2, steareth-20, steareth-21, ceteareth-20, PPG-2 methyl glucose ether distearate, ceteth-10, polysorbate 80, cetyl phosphate, potassium cetyl phosphate, diethanolamine cetyl phosphate, polysorbate 60, glyceryl stearate, PEG-100 stearate, C₂₀-C₄₀ alcohols, PEG/PPG-18/18 dimethicone, maltodextrin, sodium polyacrylate, and mixtures thereof.

3. The transdermal and/or dermal composition of claim 2, wherein the emulsifier is polysorbate 60.

4. The transdermal and/or dermal composition of claim 1, further comprising an emollient selected from cetostearyl alcohol, glycerine, glyceryl mono-stearate, and mixtures thereof.

5. The transdermal and/or dermal composition according to claim 1, wherein the composition comprises from about 0.0001% to about 20% by weight of cannabidiol.

6. The transdermal and/or dermal composition of claim 1, further comprising a higher alcohol selected from lauryl alcohol, cetyl alcohol, stearyl alcohol, behenyl alcohol, myristyl alcohol, oleyl alcohol, cetostearyl alcohol, monostearyl glycerine ether (batyl alcohol), 2-decyltetradecinol, lanolin alcohol, cholesterol, phytosterol, hexyl dodecanol, isostearyl alcohol, and octyldodecanol.

7. The transdermal and/or dermal composition according to claim 1, wherein the dehydrated mixture comprises:
i) 300 mg cannabidiol (CBD);
ii) 600 mg sunflower oil;
iii) 8 g cetostearyl alcohol;
iv) 2 g glyceryl monostearate;
v) 5 g liquid paraffin;
vi) 10 g white soft paraffin, and optionally
vii) 10 g propylene glycol monocaprylate or 10 g diethylene glycol monoethyl ether; and
wherein the aqueous phase comprises:
i) 3.6 g polysorbate 60;
ii) 30 g glycerine; and
iii) 41.7 g water.

## Patentansprüche

1. Transdermale und/oder dermale Zusammensetzung, die ein lipophiles aktives Mittel in einer Öl-in-Wasser-Emulsion umfasst, die eine wässrige Phase und eine Ölphase umfasst, wobei die Zusammensetzung erhältlich ist durch Bereitstellen einer Ölphase, die eine entwässerte Mischung umfasst, und wobei die entwässerte Mischung eine therapeutisch wirksame Menge des lipophilen aktiven Mittels und ein genießbares Öl umfasst, das langkettige Fettsäuren und/oder mittelkettige Fettsäuren umfasst, wobei das lipophile aktive Mittel Cannabidiol ist und das genießbare Öl Sonnenblumenöl ist, und wobei die entwässerte Mischung erhältlich ist durch die Schritte:
i) Kombinieren der therapeutisch wirksamen Menge des Cannabidiols mit dem Sonnenblumenöl; und
ii) Entwässern des Produkts aus Schritt (i), dadurch Herstellen der entwässerten Mischung; und Kombinieren der Ölphase mit einer wässrigen Phase, um die Öl-in-Wasser-Emulsion bereitzustellen.

2. Transdermale und/oder dermale Zusammensetzung nach Anspruch 1, die ferner einen Emulgator umfasst, wobei der Emulgator ausgewählt ist aus: Estern von Glyzerin, Estern von Propylenglykol, Fettsäureestern von Polyethylenglykol, Fettsäureestern von Polypropylenglykol, Estern von Sorbitol, Estern von Sorbitananhydriden, hydrolysierten Jojobaestern, Carbonsäure-Copolymeren, Estern und Ethern von Glukose, ethoxylierten Ethern, ethoxylierten Alkoholen, Alkylphosphaten, Polyoxyethylenfettetherphosphaten, Fettsäureamiden, Acyllactylaten, Seifen, Triethylamin-(TEA-)stearat, Diethylamin-(DEA-)oleth-3-phosphat, Polyethylenglykol-20-sorbitan-monolaurat (Polysorbat 20), Polyethylenglykol-5-sojasterol, Steareth-2, Steareth-20, Steareth-21, Ceteareth-20, PPG-2-Methylglukoseetherdistearat, Ceteth-10, Polysorbat 80, Cetylphosphat, Kaliumcetylphosphat, Diethanolamincetylphosphat, Polysorbat 60, Glycerylstearat, PEG-100-stearat, C₂₀-C₄₀-Alkoholen, PEG/PPG-18/18-dimethicon, Maltodextrin, Natriumpolyacrylat und Mischungen davon.

3. Transdermale und/oder dermale Zusammensetzung nach Anspruch 2, wobei der Emulgator Polysorbat 60 ist.

4. Transdermale und/oder dermale Zusammensetzung nach Anspruch 1, die ferner ein weichmachendes Mittel umfasst, das aus Cetylstearylalkohol, Glyzerin, Glycerylmonostearat und Mischungen davon ausgewählt ist.

5. Transdermale und/oder dermale Zusammensetzung nach Anspruch 1, wobei die Zusammensetzung etwa 0,0001 Gew.-% bis etwa 20 Gew.-% Cannabidiol umfasst.

6. Transdermale und/oder dermale Zusammensetzung nach Anspruch 1, die ferner einen höheren Alkohol umfasst, der ausgewählt ist aus Laurylalkohol, Cetylalkohol, Stearylalkohol, Behenylalkohol, Myristylalkohol, Oleylalkohol, Cetylstearylalkohol, Monostearylglyzerinether (Batylalkohol), 2-Decyltetradecinol, Lanolinalkohol, Cholesterol, Phytosterol, Hexyldodecanol, Isostearylalkohol und Octyldodecanol.

7. Transdermale und/oder dermale Zusammensetzung nach Anspruch 1, wobei die entwässerte Mischung umfasst:
i) 300 mg Cannabidiol (CBD);
ii) 600 mg Sonnenblumenöl;
iii) 8 g Cetylstearylalkohol;
iv) 2 g Glycerylmonostearat;
v) 5 g flüssiges Paraffin;
vi) 10 g weißes weiches Paraffin, und wahlweise
vii) 10 g Propylenglykolmonocaprylat oder 10 g > Diethylenglykolmonoethylether; und
wobei die wässrige Phase umfasst:
i) 3,6 g Polysorbat 60;
ii) 30 g Glyzerin; und
iii) 41,7 g Wasser.

## Revendications

1. Composition transdermique et/ou dermique comprenant un agent actif lipophile dans une émulsion huile dans l'eau comprenant une phase aqueuse et une phase huileuse, où la composition peut être obtenue en fournissant une phase huileuse comprenant un mélange déshydraté, et le mélange déshydraté comprenant une quantité thérapeutiquement efficace de l'agent actif lipophile et une huile alimentaire comprenant des acides gras en chaîne longue et/ou des acides gras en chaîne intermédiaire, dans laquelle l'agent actif lipophile est du cannabidiol et l'huile comestible est de l'huile de tournesol, et dans laquelle le mélange déshydraté peut être obtenu au moyen des étapes suivantes :
i) la combinaison de la quantité thérapeutiquement efficace du cannabidiol avec l'huile de tournesol ; et
ii) la déshydratation du produit de l'étape (i), produisant ainsi le mélange déshydraté ; et la combinaison de la phase huileuse avec une phase aqueuse pour obtenir l'émulsion huile dans l'eau.

2. Composition transdermique et/ou dermique selon la revendication 1, comprenant en outre un émulsifiant, dans laquelle l'émulsifiant est sélectionné parmi : les esters de glycérine, les esters de propylène glycol, les esters d'acides gras de polyéthylène glycol, les esters d'acides gras de polypropylène glycol, les esters de sorbitol, les esters d'anhydride de sorbitane, les esters de jojoba hydrolysés, les copolymères d'acide carboxylique, les esters et les éthers de glucose, les éthers éthoxylés, les alcools éthoxylés, les alkylphosphates, les phosphates d'éther gras de polyoxyéthylène, les amides d'acides gras, les lactylates d'acyle, les savons, le stéarate de triéthylamine (TEA), l'oleth-3 phosphate de diéthylamine (DEA), le monolaurate de polyéthylène glycol sorbitane 20 (polysorbate 20), le stérol de soja de polyéthylène glycol 5, le stéareth-2, le stéareth-20, le stéareth-21, le cétéareth-20, le distéarate d'éther de méthylglucose de PPG-2, le céteth-10, le polysorbate 80, le cétylphosphate, le cétylphosphate de potassium, le cétylphosphate de diéthanolamine, le polysorbate 60, le stéarate de glycéryle, le stéarate de PEG-100, les alcools en C₂₀-C₄₀, le diméthicone de PEG/PPG-18/18, la maltodextrine, le polyacrylate de sodium et leurs mélanges.

3. Composition transdermique et/ou dermique selon la revendication 2, dans laquelle l'émulsifiant est le polysorbate 60.

4. Composition transdermique et/ou dermique selon la revendication 1, comprenant en outre un émollient sélectionné parmi l'alcool cétostéarylique, la glycérine, le monostéarate de glycéryle et leurs mélanges.

5. Composition transdermique et/ou dermique selon la revendication 1, dans laquelle la composition comprend environ de 0,0001 % à environ 20 % en poids de cannabidiol.

6. Composition transdermique et/ou dermique selon la revendication 1, comprenant en outre un alcool supérieur sélectionné parmi l'alcool laurylique, l'alcool cétylique, l'alcool stéarylique, l'alcool béhénylique, l'alcool myristique, l'alcool d'oléyle, l'alcool cétostéarylique, l'éther de glycérine monostéarylique (alcool de batyle), le 2-décyltétradécinol, l'alcool de lanoline, le cholestérol, le phytostérol, l'hexyldodécanol, l'alcool isostéarylique et l'octyldodécanol.

7. Composition transdermique et/ou dermique selon la revendication 1, dans laquelle le mélange déshydraté comprend :
i) 300 mg de cannabidiol (CBD) ;
ii) 600 mg d'huile de tournesol ;
iii) 8 g d'alcool cétostéarylique ;
iv) 2 g de monostéarate de glycéryle ;
v) 5 g de paraffine liquide ;
vi) 10 g de paraffine molle blanche, et optionnellement
vii) 10 g de monocaprylate de propylène glycol ou 10 g d'éther monoéthylique de diéthylène glycol ; et
dans laquelle la phase aqueuse comprend :
i) 3,6 g de polysorbate 60 ;
ii) 30 g de glycérine ; et
iii) 41,7 g d'eau.
